# EUROPEAN PATENT APPLICATION

(11) **EP 4 431 596 A1**
(43) Date of publication of application: **18.09.2024**
(21) Application number: 22892867.7
(22) Date of filing: 10.11.2022
(51) Int. Cl.: C12N 5/071, A61K 35/30, A61P 27/02, C12N 1/04

(54) **CRYOPRESERVATION PREPARATION FOR CORNEAL ENDOTHELIAL CELLS AND METHOD FOR PRODUCING SAID CRYOPRESERVATION PREPARATION**

(30) Priority: 11.11.2021 JP 2021184246
(71) Applicant: The Doshisha, Kyoto 602-8580 (JP); ActualEyes Inc., Kyotanabe-shi, Kyoto 610-0332 (JP)
(72) Inventor: KOIZUMI, Noriko, Kyotanabe-shi, Kyoto 610-0394 (JP); OKUMURA, Naoki, Kyotanabe-shi, Kyoto 610-0394 (JP); MATSUOKA, Yasushi, Kyotanabe-shi, Kyoto 610-0332 (JP)
(74) Representative: Vossius & Partner Patentanwälte Rechtsanwälte mbB
(86) International application number: PCT/JP2022/041960
(87) International publication number: WO 2023/085369

(57) **Abstract**

The present disclosure provides a method for cryopreserving corneal endothelial cells and/or corneal endothelial-like cells, and a freezing prepartaion for corneal endothelial cells and/or corneal endothelial-like cells. The present disclosure provides a method for preserving corneal endothelial cells and/or corneal endothelial-like cells, wherein the method includes: a freezing step for freezing unfrozen corneal endothelial cells and/or corneal endothelial-like cells, the freezing step including at least one stage in which the temperature is reduced at a rate of less than 1°C/min when the temperature is changed from the unfrozen temperature to the freezing target temperature; and, if necessary, a step for maintaining the corneal endothelial cells and/or the corneal endothelial-like cells in a frozen state.

## Description

### [Technical Field]

The present disclosure relates to a technology for a cryopreservation formulation of corneal endothelial cells and a method for producing the formulation as well as an applied technology such as a treatment using them.

### [Background Art]

When corneal endothelial cells are damaged, the cornea becomes cloudy, resulting in severe visual impairment due to bullous keratopathy. Although the only treatment for bullous keratopathy is corneal transplantation, there are problems such as a shortage of donors and rejection. Therefore, development of a new regenerative medicine treatment is desired. Doshisha University has established treatment for corneal endothelial regeneration by proliferating corneal endothelial cells isolated from the cornea of a donor in the presence of a Rho-associated coiled-coil forming kinase (ROCK) inhibitor and injecting the cells into many patients. However, limitation of a period of time during which the cells can be preserved while keeping their quality has caused a major problem in supplying the cells throughout Japan and around the world. Generally, cells can be cryopreserved in a preservation solution containing 10% dimethyl sulfoxide (DMSO), but, in the case of regenerative medicine, it has been desired to freeze the cells with a reduced concentration of DMSO due to concerns about toxicity of DMSO to the cells and irritation when administered to the eye.

In addition, since a frozen cell formulation to be used for regenerative medicine contains at least 7% or more DMSO, when it is administered to a patient, administration of a high concentration of DMSO to the patient has been avoided by diluting the formulation with saline immediately prior to administration or administering the formulation at an extremely slow rate, for example, in the case of intravenous infusion due to a concern about toxicity of DMSO to the patient.

Because corneal endothelial cell infusion therapy requires administration of a cell suspension with a high concentration in a low dose of 400 µL or less to the anterior chamber of the eye of a patient, the suspension cannot be diluted prior to administration. Therefore, there is a need for a cell cryopreservation formulation with a reduced concentration of or free from DMSO.

### [Summary of Invention]

### [Solution to Problem]

The present inventors have examined in detail, for example, temperature conditions upon cryopreservation and found that a temperature drop at a slower rate than -1°C/min allows viability of corneal endothelial cells to be maintained high in a cryopreservation solution with a reduced concentration (e.g., less than 7%) of or free from DMSO. The present inventors further found that cell viability is increased when cells are initially decreased in temperature at a slow rate and then frozen at a faster cooling rate. Thus, the present disclosure provides a method for freezing corneal endothelial cells in a cryopreservation solution with a reduced concentration of or free from DMSO and a method for producing a frozen cell formulation that can be administered directly to a patient.

The invention of the present application provides, for example, the following items.

### (Item 1)

A method for preserving corneal endothelial cells and/or corneal endothelium-like cells, the method including:
freezing the corneal endothelial cells and/or the corneal endothelium-like cells in a non-frozen state, the freezing including at least one step of decreasing a temperature at a rate of less than 1°C per minute when changing the temperature from a non-frozen temperature to a freezing target temperature; and
optionally maintaining the corneal endothelial cells and/or the corneal endothelium-like cells in a frozen state.

### (Item 2)

The method according to the above item, the method includes maintaining the corneal endothelial cells and/or the corneal endothelium-like cells in a frozen state.

### (Item 3)

The method according to any one of the above items, in which maintaining the corneal endothelial cells and/or the corneal endothelium-like cells in a frozen state includes maintaining the corneal endothelial cells and/or the corneal endothelium-like cells at a freeze maintenance temperature.

### (Item 4)

The method according to any one of the above items, in which the freeze maintenance temperature is a temperature in a range of about -80°C to about -10°C.

### (Item 5)

The method according to any one of the above items, in which the freeze maintenance temperature is a temperature in a range of about -196°C to about -10°C.

### (Item 6)

The method according to any one of the above items, in which the freeze maintenance temperature is a temperature of about -30°C or less.

### (Item 7)

The method according to any one of the above items, in which the corneal endothelial cells and/or the corneal endothelium-like cells are decreased in temperature at a rate of about 0.1°C to about 0.9°C per minute from the non-frozen temperature.

### (Item 8)

The method according to any one of the above items, in which the corneal endothelial cells and/or the corneal endothelium-like cells are decreased in temperature at a rate of about 0.2°C to about 0.8°C per minute from the non-frozen temperature.

### (Item 9)

The method according to any one of the above items, in which the corneal endothelial cells and/or the corneal endothelium-like cells are decreased in temperature at a rate of about 0.7°C per minute or less from the non-frozen temperature.

### (Item 10)

The method according to any one of the above items, in which the corneal endothelial cells and/or the corneal endothelium-like cells are decreased in temperature at a rate of about 0.2°C to about 0.7°C per minute from the non-frozen temperature.

### (Item 11)

The method according to any one of the above items, in which the non-frozen temperature is a temperature in a range of about 0°C to about 42°C.

### (Item 12)

The method according to any one of the above items, in which the non-frozen temperature is a temperature in a range of about 0°C to about 37°C.

### (Item 13)

The method according to any one of the above items, in which the non-frozen temperature is a temperature in a range of about 4°C to about 23°C.

### (Item 14)

The method according to any one of the above items, in which freezing includes at least one step of decreasing a temperature at a rate of less than 1°C per minute in at least a portion of a temperature range of about -20°C ± 10°C.

### (Item 15)

The method according to any one of the above items, in which freezing includes at least one step of maintaining a temperature in a range of about -20°C ± 10°C for a period of time or longer.

### (Item 16)

The method according to any one of the above items, in which the corneal endothelial cells and/or the corneal endothelium-like cells are preserved in a preservation solution containing less than about 7% DMSO.

### (Item 17)

The method according to any one of the above items, in which the corneal endothelial cells and/or the corneal endothelium-like cells are preserved in a preservation solution containing about 5% or less DMSO.

### (Item 18)

The method according to any one of the above items, in which the corneal endothelial cells and/or the corneal endothelium-like cells are preserved in a preservation solution containing about 2% or less DMSO.

### (Item 19)

The method according to any one of the above items, in which the corneal endothelial cells and/or the corneal endothelium-like cells are preserved in a preservation solution free from DMSO.

### (Item 20)

The method according to any one of the above items, in which freezing includes freezing the corneal endothelial cells and/or the corneal endothelium-like cells in a presence of a ROCK inhibitor.

### (Item 21)

The method according to any one of the above items, in which freezing includes decreasing a temperature at a first rate to a first target temperature and decreasing the temperature at a second rate from the first target temperature to a second target temperature, the first rate being less than 1°C per minute and slower than the second rate.

### (Item 22)

The method according to any one of the above items, in which freezing further includes decreasing a temperature to the first target temperature and then maintaining the temperature at the first target temperature.

### (Item 23)

The method according to any one of the above items, in which the first target temperature is a temperature in a range of about -20°C to about -5°C.

### (Item 24)

The method according to any one of the above items, in which the first target temperature is a temperature in a range of about -15°C to about -10°C.

### (Item 25)

The method according to any one of the above items, in which the second target temperature is a temperature of about - 20°C or less.

### (Item 26)

The method according to any one of the above items, in which the second target temperature is a temperature in a range of about -196°C to about -80°C.

### (Item 27)

The method according to any one of the above items, in which the first rate is a rate of about 0.5°C to about 0.05°C per minute.

### (Item 28)

The method according to any one of the above items, in which the first rate is a rate of about 0.3°C to about 0.1°C per minute.

### (Item 29)

The method according to any one of the above items, in which the second rate is a rate of about 0.5 to about 5°C per minute.

### (Item 30)

The method according to any one of the above items, in which the second rate is a rate of about 1 to about 3°C per minute.

### (Item 31)

A method for producing a frozen formulation of corneal endothelial cells and/or corneal endothelium-like cells, the method including:
freezing the corneal endothelial cells and/or the corneal endothelium-like cells in a non-frozen state optionally mixed with a pharmaceutically acceptable component to thereby produce a frozen formulation, the freezing including at least one step of decreasing a temperature at a rate of less than 1°C per minute when changing the temperature from a non-frozen temperature to a freezing target temperature; and
optionally maintaining the frozen formulation of the corneal endothelial cells and/or the corneal endothelium-like cells in a frozen state.

### (Item 32)

The method according to any one of the above items, further including one or more features described in the method according to any one or more of items 2 to 30.

### (Item 33)

A frozen formulation of corneal endothelial cells and/or corneal endothelium-like cells produced by the method according to any one of the above items.

### (Item 34)

The frozen formulation according to any one of the above items, in which the frozen formulation includes about 1 × 10⁵ to about 3 × 10⁶ corneal endothelial cells and/or corneal endothelium-like cells.

### (Item 35)

The frozen formulation according to any one of the above items, in which the frozen formulation has a volume of about 50 µL to about 600 µL.

### (Item 36)

The frozen formulation according to any one of the above items, in which the frozen formulation is administered in a volume of about 50 µL to about 350 µL per dose.

### (Item 37)

A device for preserving corneal endothelial cells and/or corneal endothelium-like cells, the device including:
a housing/preserver that contains a container for containing the corneal endothelial cells and/or the corneal endothelium-like cells;
a temperature controller that gives an instruction to control a temperature of the corneal endothelial cells and/or the corneal endothelium-like cells in the container contained in the housing/preserver; and
a temperature regulator that is able to regulate the temperature in the housing/preserver based on the instruction from the temperature controller,
the temperature controller being able to give an instruction to control the temperature so as to include at least one step of changing the temperature at a rate of less than 1°C per minute when decreasing the temperature from a non-frozen temperature to a freezing target temperature and optionally to maintain the corneal endothelial cells and/or the corneal endothelium-like cells in a frozen state.

### (Item 38)

A program for causing a computer to execute a method for preserving corneal endothelial cells and/or corneal endothelium-like cells in a device, the device including:
a housing/preserver that contains a container for containing the corneal endothelial cells and/or the corneal endothelium-like cells;
a temperature controller that gives an instruction to control a temperature of the corneal endothelial cells and/or the corneal endothelium-like cells in the container contained in the housing/preserver; and
a temperature regulator that is able to regulate the temperature in the housing/preserver based on the instruction from the temperature controller,
the program causing the temperature controller to execute:
   controlling the temperature so as to include at least one step of changing the temperature at a rate of less than 1°C per minute when decreasing the temperature from a non-frozen temperature to a freezing target temperature; and
   optionally maintaining the corneal endothelial cells and/or the corneal endothelium-like cells in a frozen state.

### (Item 39)

A recording medium storing a program for causing a computer to execute a method for preserving corneal endothelial cells and/or corneal endothelium-like cells in a device, the device including:
a housing/preserver that contains a container for containing the corneal endothelial cells and/or the corneal endothelium-like cells;
a temperature controller that gives an instruction to control a temperature of the corneal endothelial cells and/or the corneal endothelium-like cells in the container contained in the housing/preserver; and
a temperature regulator that is able to regulate the temperature in the housing/preserver based on the instruction from the temperature controller,
the program causing the temperature controller to execute:
   controlling the temperature so as to include at least one step of changing the temperature at a rate of less than 1°C per minute when decreasing the temperature from a non-frozen temperature to a freezing target temperature; and
   optionally maintaining the corneal endothelial cells and/or the corneal endothelium-like cells in a frozen state.

### (Item 40)

A frozen formulation including:
less than 7% DMSO; and
corneal endothelial cells and/or corneal endothelium-like cells.

### (Item 41)

A frozen formulation including:
less than 7% DMSO; and
corneal endothelial cells and/or corneal endothelium-like cells,
the frozen formulation being able to be administered directly to an eye after thawing.

### (Item 42)

A frozen formulation including:
less than 7% DMSO; and
corneal endothelial cells and/or corneal endothelium-like cells,
the DMSO and the corneal endothelial cells and/or the corneal endothelium-like cells being in a slow frozen state.

### (Item 43)

A frozen formulation including:
less than 7% DMSO;
corneal endothelial cells and/or corneal endothelium-like cells; and
a saline component,
the DMSO, the corneal endothelial cells and/or the corneal endothelium-like cells, and the saline component being in a frozen state.

### (Item 44)

A frozen formulation including:
less than 7% DMSO;
corneal endothelial cells and/or corneal endothelium-like cells; and
a medium component,
the DMSO, the corneal endothelial cells and/or the corneal endothelium-like cells, and the medium component being in a frozen state.

### (Item 45)

A post-thaw long-term stable frozen cell formulation including:
less than 7% DMSO; and
corneal endothelial cells and/or corneal endothelium-like cells.

### (Item 46)

The frozen formulation according to any one of the above items, in which the frozen formulation includes about 5% or less DMSO.

### (Item 47)

The frozen formulation according to any one of the above items, in which the frozen formulation includes about 2% or less DMSO.

### (Item 48)

The frozen formulation according to any one of the above items, in which the frozen formulation is free from DMSO.

### (Item 49)

The frozen formulation according to any one of the above items, further including a ROCK inhibitor.

### (Item 50)

The frozen formulation according to any one of the above items, in which the ROCK inhibitor is Y-27632.

### (Item 51)

A frozen formulation including:
a ROCK inhibitor; and
corneal endothelial cells and/or corneal endothelium-like cells,
the ROCK inhibitor and the corneal endothelial cells and/or the corneal endothelium-like cells being in a frozen state.

### (Item 52)

A frozen formulation including:
a ROCK inhibitor; and
corneal endothelial cells and/or corneal endothelium-like cells; and
a saline component,
the ROCK inhibitor, the corneal endothelial cells and/or the corneal endothelium-like cells, and the saline component being in a frozen state.

### (Item 53)

A frozen formulation including:
a ROCK inhibitor;
corneal endothelial cells and/or corneal endothelium-like cells; and
a medium component,
the ROCK inhibitor, the corneal endothelial cells and/or the corneal endothelium-like cells, and the medium component being in a frozen state.

### (Item 54)

A post-thaw long-term stable frozen cell formulation including:
corneal endothelial cells and/or corneal endothelium-like cells; and
a ROCK inhibitor.

### (Item 55)

The formulation according to any one of the above items, in which the corneal endothelial cells and/or the corneal endothelium-like cells have at least 80% viability for at least 6 hours at room temperature after thawing.

### (Item 56)

A frozen formulation including:
corneal endothelial cells and/or corneal endothelium-like cells; and
a ROCK inhibitor,
the corneal endothelial cells and/or the corneal endothelium-like cells and the ROCK inhibitor being in a slow frozen state, and
the formulation not inhibiting engraftment and survival in vivo of the corneal endothelial cells and/or the corneal endothelium-like cells administered after thawing.

### (Item 57)

A frozen formulation including:
less than 7% DMSO;
a ROCK inhibitor; and
corneal endothelial cells and/or corneal endothelium-like cells,
the DMSO, the ROCK inhibitor, and the corneal endothelial cells and/or the corneal endothelium-like cells being in a slow frozen state.

### (Item 58)

The formulation according to any one of the above items, in which the ROCK inhibitor is Y-27632.

### (Item 59)

The formulation according to any one of the above items, in which the formulation includes less than about 7% DMSO.

### (Item 60)

The formulation according to any one of the above items, in which the formulation includes about 5% or less DMSO.

### (Item 61)

The formulation according to any one of the above items, in which the formulation includes about 2% or less DMSO.

### (Item 62)

The formulation according to any one of the above items, in which the formulation is free from DMSO.

### (Item 63)

The formulation according to any one of the above items, in which the formulation includes the cells in a slow-frozen state.

### (Item 64)

The formulation according to any one of the above items, in which formulation is frozen by decreasing a temperature at a rate of less than 1°C per minute from a non-frozen temperature.

### (Item 65)

The formulation according to any one of the above items, in which the corneal endothelial cells and/or the corneal endothelium-like cells are used for a cell infusion therapy.

### (Item 66)

The formulation according to any one of the above items, in which the frozen formulation is administered after thawing without further processing or culturing.

### (Item 67)

The formulation according to any one of the above items, in which the formulation includes about 1 × 10⁵ to about 3 × 10⁶ corneal endothelial cells and/or corneal endothelium-like cells.

### (Item 68)

The formulation according to any one of items 40 to 67, in which the frozen formulation has a volume of about 50 µL to about 600 µL.

### (Item 69)

The formulation according to any one of the above items, in which the formulation is administered in a volume of about 50 µL to about 350 µL per dose.

### (Item 70)

A frozen formulation kit including:
a container that contains a frozen formulation including corneal endothelial cells and/or corneal endothelium-like cells in a frozen state; and
a housing that contains the container while maintaining it in a frozen state.

### (Item 71)

The frozen formulation kit according to item 70, in which the frozen formulation is the formulation according to any one of the above items.

### (Item 72)

A frozen formulation kit including:
the formulation according to any one of the above items;
a container that contains the formulation; and
a housing that contains the container while maintaining the formulation in a frozen state.

### (Item 73)

A frozen formulation kit including:
a container; and
a housing that contains the container,
the container being used to contain the formulation according to any one of the above items, and the housing being used to maintain the formulation in a frozen state.

### (Item 74)

A frozen formulation kit including:
a container that contains a frozen formulation including a ROCK inhibitor and corneal endothelial cells and/or corneal endothelium-like cells both in a frozen state; and
a housing that contains the container while maintaining it in a frozen state.

### (Item 75)

A use of a kit, the kit including:
a container; and
a housing that contains the container,
the container being used to contain the formulation according to any one of the above items, and the housing being used to maintain the formulation in a frozen state.

### (Item 76)

A method for transporting and/or preserving a formulation according to any one of the above items, the method including:
placing the formulation in a container of a kit including the container and a housing that contains the container, and
maintaining the formulation in the kit in a frozen state.

### (Item 77)

A method for performing corneal endothelial cell injection therapy, the method including:
providing corneal endothelial cells and/or corneal endothelium-like cells suitable for the cell infusion therapy;
freezing the corneal endothelial cells and/or the corneal endothelium-like cells, the freezing including at least one step of decreasing a temperature of the corneal endothelial cells and/or the corneal endothelium-like cells at a rate of less than 1°C per minute from a non-frozen temperature;
maintaining the corneal endothelial cells and/or the corneal endothelium-like cells in a frozen state and optionally transporting them to the infusion therapy;
thawing the corneal endothelial cells and/or the corneal endothelium-like cells; and
administering the corneal endothelial cells and/or the corneal endothelium-like cells to a subject.

### (Item 1A)

A method for preserving corneal endothelial cells and/or corneal endothelium-like cells, the method including:
freezing the corneal endothelial cells and/or the corneal endothelium-like cells in a non-frozen state, the freezing including decreasing a temperature at a first rate to a first target temperature and decreasing the temperature at a second rate from the first target temperature to a second target temperature; and
optionally maintaining the corneal endothelial cells and/or the corneal endothelium-like cells in a frozen state,
the first rate being less than 1°C per minute and slower than the second rate.

### (Item 2A)

The method according to any one of the above items, in which freezing further includes decreasing a temperature to the first target temperature and then maintaining the temperature at the first target temperature.

### (Item 3A)

The method according to any one of the above items, in which the first target temperature is a temperature in a range of about -20°C to about -5°C.

### (Item 4A)

The method according to any one of the above items, in which the first target temperature is a temperature in a range of about -15°C to about -10°C.

### (Item 5A)

The method according to any one of the above items, in which the second target temperature is a temperature of about - 20°C or less.

### (Item 6A)

The method according to any one of the above items, in which the second target temperature is a temperature in a range of about -196°C to about -80°C.

### (Item 7A)

The method according to any one of the above items, in which the first rate is a rate of about 0.5°C to about 0.05°C per minute.

### (Item 8A)

The method according to any one of the above items, in which the first rate is a rate of about 0.3°C to about 0.1°C per minute.

### (Item 9A)

The method according to any one of the above items, in which the second rate is a rate of about 0.5 to about 5°C per minute.

### (Item 10A)

The method according to any one of the above items, in which the second rate is a rate of about 1 to about 3°C per minute.

In the present disclosure, it is intended that one or more of the above features may be provided in additional combinations in addition to the explicit combinations. Those skilled in the art will appreciate further embodiments and advantages of the present disclosure upon reading and understanding the following detailed description, if necessary.

### [Advantageous Effects of Invention]

The present disclosure provides a frozen formulation of corneal cells that can be administered directly to the eye after thawing. The present invention can also provide corneal endothelial cells throughout Japan and abroad.

### [Brief Description of Drawings]

[FIG. 1] Figure 1 shows an overview of Example 1;
[FIG. 2] Figure 2 shows photographs of morphology of cultured cells used in Example 1;
[FIG. 3] Figure 3 shows a graph comparing cell viability after being preserved in cryopreservation solutions with 4% human serum albumin and 10% glycerin serving as base components and different concentrations of DMSO and thawed;
[FIG. 4] Figure 4 shows a graph comparing cell viability after being preserved in cryopreservation solutions with 4% human serum albumin and 10% polyethylene glycol serving as base components and different concentrations of DMSO and thawed;
[FIG. 5] Figure 5 shows data comparing viability after thawing cells frozen at a cooling rate of -1°C/min, -0.7°C/min, or -0.5°C/min from 23°C. Error bars indicate mean ± SD. Statistical significance is based on Dunnett's t-test (vs unfrozen) (n = 3, **p < 0.05);
[FIG. 6] Figure 6 shows data comparing cell densities on day 7 after freezing cells at a cooling rate of -1°C/min or - 0.5°C/min from 4°C, preserving, and then seeding;
[FIG. 7] Figure 7 shows an overview of Example 2;
[FIG. 8] Figure 8 shows phase contrast micrographs of cells cultured in a medium supplemented with 10% or 5% DMSO;
[FIG. 9] Figure 9 shows phase contrast micrographs of cells cultured in a medium with 2% or without DMSO;
[FIG. 10] Figure 10 shows a graph showing results of viability of cells after reseeded and collected. From left to right for each group, 10%, 5%, 2%, and 0% DMSO are shown;
[FIG. 11] Figure 11 shows an overview of Example 3;
[FIG. 12] Figure 12 shows phase contrast microscopy images of cells frozen in Cryostor CS10 containing 10% DMSO, left to stand at room temperature for 0 h, 30 min, 1 h, 3 h, 6 h, or 24 h, then reseeded into T25 culture flasks, and 24 hours later photographed in culture;
[FIG. 13] Figure 13 shows phase contrast microscopy images of cells frozen in Cryostor CS5 containing 5% DMSO, left to stand at room temperature for 0 h, 30 min, 1 h, 3 h, 6 h, or 24 h, then reseeded into T25 culture flasks, and 24 hours later photographed in culture;
[FIG. 14] Figure 14 shows phase contrast microscopy images of cells frozen in Cryostor CS2 containing 2% DMSO, left to stand at room temperature for 0 h, 30 min, 1 h, 3 h, 6 h, or 24 h, then reseeded into T25 culture flasks, and 24 hours later photographed in culture;
[FIG. 15] Figure 15 shows a graph showing results of viability of cells after reseeded and collected. From left to right for each group, CS10, CS5, and CS2 are shown;
[FIG. 16] Figure 16 shows viability and cell recovery rate of cells after being preserved in a cold box for 5 days and thawed. Error bars indicate mean ± SD. Statistical significance is based on Student's t-test (n = 3);
[FIG. 17] Figure 17 shows micrographs of cells cultured for 2 days after being preserved in a cold box;
[FIG. 18] Figure 18 shows photographs of a rabbit eye injected with cells after being preserved in a cold box;
[FIG. 19] Figure 19 shows photographs of immunohistochemical staining for CD166 in the corneal endothelium on day 1 after cell injection;
[FIG. 20] Figure 20 shows photographs of immunohistochemical staining for ZO-1 and Na/K ATPase on day 1 after cell injection;
[FIG. 21] Figure 21 shows photographs of immunohistochemical staining for CD166, ZO-1, and Na/K ATPase in the corneal endothelium on day 5 after cell injection;
[FIG. 22] Figure 22 shows an overview of cryopreservation in cryopreservation solutions containing known components of Example 6;
[FIG. 23] Figure 23 shows a graph comparing cell viability after being frozen in cryopreservation solutions with 4% human serum albumin and 10% glycerin serving as base components and different concentrations of DMSO at a cooling rate of -1°C/min, -0.7°C/min, -0.5°C/min, or -0.2°C/min and thawed;
[FIG. 24] Figure 24 shows an overview of cryopreservation in commercially available cryopreservation solutions of Example 6;
[FIG. 25] Figure 25 shows cell viability and recovery rate after frozen at a cooling rate of -0.7°C/min from 4°C in commercially available cryopreservation solutions and thawed;
[FIG. 26] Figure 26 shows data comparing cell viability after frozen at a cooling rate of -1°C/min or -0.7°C/min from 4°C in commercially available cryopreservation solutions and thawed;
[FIG. 27] Figure 27 shows data comparing cell viability after frozen at a cooling rate of -0.5°C/min or -0.2°C/min from 4°C in commercially available cryopreservation solutions and thawed;
[FIG. 28] Figure 28 shows cell viability of cells preserved in Example 7;
[FIG. 29] Figure 29 shows temperature transition when cooled to -80°C at -0.5°C/min;
[FIG. 30] Figure 30 shows the temperature transition when cooled to -10°C at -0.5°C/min, maintaining the temperature at - 10°C for 110 minutes, and then cooled to -80°C at -1.0°C/min; and
[FIG. 31] Figure 31 shows temperature transition when cooled to -10°C at -0.1°C/min and then to -80°C at -1.0°C/min.

### [Description of Embodiments]

The present disclosure will be described. It should be understood that singular expressions also include the concept of their plural forms throughout the specification, unless otherwise noted. Thus, it should be understood that singular articles (e.g., "a", "an", "the", etc. in English) also include the concept of their plural forms, unless otherwise noted. It should also be understood that terms used herein are to be used in the sense normally used in the field, unless otherwise noted. Therefore, unless otherwise defined, all technical and scientific terms used herein have the same meaning as generally understood by those skilled in the art to which the present disclosure pertains. In the event of a conflict, this specification (including definitions) shall prevail. As used herein, the term "about" means ± 10% of the value that follows. The symbol "%" denoting composition means w/w% when referring to DMSO and human serum albumin (HSA) and v/v% when referring to glycerin and polyethylene glycol, unless otherwise indicated.

### (Definitions)

As used herein, the phrase "corneal endothelial cells" is used in the usual sense used in the field. The cornea is one of lamellar tissues that make up the eye, is transparent, and is located closest to the outside world. In humans, the cornea is composed of five layers: the corneal epithelium, the Bowman's membrane, the lamina propria, the Descemet's membrane (basement membrane of corneal endothelium), and the corneal endothelium from the outside (body surface). Unless otherwise specified, portions of the cornea other than the epithelium and the endothelium are sometimes collectively referred to as the "corneal stroma" and are so referred to herein. As used herein, the term "HCEC" is an abbreviation for human corneal endothelial cells.

As used herein, the phrase "corneal endothelium-like cells" refers to cells differentiated from stem cells, such as iPS cells, that have substantially the same functions as corneal endothelial cells. Methods of differentiating stem cells, such as embryonic stem cells (ES cells) and induced pluripotent stem cells (iPS cells), into corneal endothelium-like cells are well known in the field (McCabe et al., PLoS One. 2015 Dec 21; 10 (12): e0145266; Ali et al., Invest Ophthalmol Vis Sci. 2018 May 1; 59 (6): 2437-2444). In a typical example, briefly, iPS cells are seeded in 35 mm Matrigel-coated plates (Corning) at a 1:12 dilution on day 0 using a cell dissociation buffer (Life Technologies) (a plate with 80% confluent is divided into 12 plates). The iPS cells are grown in a medium (mTeSR1; STEMCELL Technologies Inc.) for 4 days. On day 4, the mTeSR1 medium was replaced with a Smad inhibitor medium containing 500 ng/mL human recombinant Noggin (R&D Systems, Minneapolis, MN, USA) and 10 µM SB431542 (MilliporeSigma) in a basal medium composed of 80% DMEM-F12 (Life Technologies), 20% KSR (Life Technologies), 1% non-essential amino acids (Life Technologies), 1 mM L-glutamine (STEMCELL Technologies, Inc.), 0.1 mM β-mercaptoethanol (MilliporeSigma), and 8 ng/mL βFGF (MilliporeSigma). On day 6, the Smad inhibitor medium was replaced with a corneal medium containing 0.1 × B27 supplement (Life Technologies), 10 ng/mL recombinant human platelet-derived growth factor-BB (PDGF-BB; PeproTech, Rocky Hill, NJ, USA) and 10 ng/mL recombinant human Dickkopf-related protein-2 (DKK-2; R&D Systems) in a basal medium composed of 80% DMEM-F12 (Life Technologies), 20% KSR (Life Technologies), 1% non-essential amino acids (Life Technologies), 1 mM L-glutamine (STEMCELL Technologies, Inc.), 0.1 mM β-mercaptoethanol (MilliporeSigma), and 8 ng/mL βFGF (MilliporeSigma). On day 7, differentiating CECs are transferred to a new Matrigel-coated plate (35 mm) and grown in a corneal medium for an additional 13 days. Differentiated CECs are collected on day 20. The above-mentioned example is a typical example, and those skilled in the art may use other methods well known in the field (Fukuta et al., PLoS One. 2014 Dec 2; 9 (12): e112291; Hayashi et al., Nature. 2016 Mar 17; 531 (7594): 376-80). In addition, those skilled in the art can produce corneal endothelium-like cells by adjusting conditions of methods well known in the art as appropriate.

The phrases "corneal endothelial cells" and "corneal endothelium-like cells" may contain a magnetic substance (e.g., iron). For example, when corneal endothelial cells containing a magnetic material are injected into the anterior chamber, magnetic force can encourage the cells to be attracted and adhered to the inner cornea (e.g., Descemet's membrane) (Patel et al., Invest Ophthalmol Vis Sci. 2009 May; 50(5): 2123-31; Mimura et al., Exp Eye Res. 2003 Jun; 76(6): 745-51; and Mimura et al., Exp Eye Res. 2005 Feb; 80(2): 149-57). The phrase "magnetic substance" refers to a substance that is magnetized by a magnetic field, such as iron, cobalt, nickel, and ferrite.

As used herein, the term "preservation" of cells means storing the cells in a container for a period of time for any purpose (e.g., cell infusion therapy or transportation therefor) without proliferating them, but with maintaining their function in the container. The preservation is different from "culture" which is intended to proliferate cells. Furthermore, the preservation does not mean transferring cells into a syringe, etc. immediately prior to administration, or temporarily holding cells in a container for prior preparation prior to administration. The term "cryopreservation" means preservation in a frozen state.

As used herein, the phrase "non-frozen temperature" refers to a temperature at which freezing does not occur when maintained at that temperature, and the phrase "freezing target temperature" refers to a target temperature for freezing corneal endothelial cells and/or corneal endothelium-like cells in a freezing process in the method of the present disclosure. The phrase "freeze maintenance temperature" refers to a temperature at which frozen corneal endothelial cells and/or corneal endothelium-like cells are maintained in a frozen state for a period of time. The freeze maintenance temperature may be varied as long as an object to be frozen such as cells of interest can remain frozen.

As used herein, the phrase "slow-frozen state" refers to a state of being frozen by a freezing process that includes at least one step of decreasing temperature at a rate of less than 1°C per minute.

As used herein, the phrase "post-thaw long-term stability" refers to maintaining at least 80% cell viability for at least 6 hours when frozen cells are thawed and then maintained at room temperature.

As used herein, the phrase "frozen formulation" refers to a formulation that is preserved in a frozen state and, after thawed, is in a form suitable for use or a form for prior preparation. The phrase "prior preparation" refers to preparation of a formulation suitable for use by adding an agent thereto or diluting it with a solvent immediately prior to administration.

As used herein, the term "processing" means, when referring to a cell or cell population, a specific operation by which any state or property of the cell or cell population is changed, preferably an operation that changes a property of the cell population such as addition of an agent, treatment with an agent, or isolation of a specific cell, or an operation such as alternation of cell density, dilution with a solvent, or concentration.

As used herein, the phrase "constant temperature" means within ± 1°C of a set temperature.

As used herein, the phrase "symptom, disorder, or disease of the corneal endothelium" refers to any symptom, disorder, or disease that occurs in the corneal endothelium. Examples of the symptom, disorder, or disease of the corneal endothelium include, but are not limited to, Fuchs' endothelial dystrophy, a post-transplant corneal disorder, corneal endotheliitis, trauma, a post-ophthalmic surgery disorder, a post-ophthalmic laser surgery disorder, aging, posterior polymorphous dystrophy (PPD), congenital hereditary endothelial dystrophy (CHED), and an idiopathic corneal endothelial disorder.

As used herein, the term "subject" refers to an object to be administered with a formulation of the present disclosure, and the subject may be a mammal (e.g., human, mouse, rat, hamster, rabbit, cat, dog, cow, horse, sheep, monkey, etc.), with a primate, especially human being preferred.

As used herein, the term "kit" refers to a unit in which parts to be provided (e.g., a test agent, a diagnostic agent, a therapeutic agent, an antibody, a label, an instruction, etc.) are provided usually in two or more compartments. Such a kit is preferred for the purpose of providing a composition that should not be provided in a mixture for stability reason, etc. and preferably is mixed immediately prior to use. Alternatively, the kit is preferred when providing a compound that is unstable in solution and needs to be subjected to prior preparation by dissolving a lyophilized powder in an appropriate solvent immediately before use. It is advantageous that such a kit preferably has a direction or instruction that describes how to use the parts to be provided (e.g., a test agent, a diagnostic agent, a therapeutic agent) or how a reagent should be processed.

As used herein, the term "program" is used in the usual sense used in the field and is an ordered description of processing to be executed by a computer, and is treated as a "product" under the patent law in Japan. All computers operate according to a program. In modern computers, a program is represented as data in the broad sense and is stored on a recording medium or storage device.

As used herein, the phrase "recording medium" is a recording medium that stores a program for executing the method of this disclosure, and the recording medium may be any medium as long as it can record the program. For example, the recording medium may be a ROM, an HDD, or a magnetic disk that can be internally enclosed, or an external storage device such as a flash memory, for example, a USB memory, etc., but not limited thereto.

As used herein, the term "system" refers to a configuration that executes the method or program of the present disclosure, and essentially means a scheme or organization for carrying out a purpose and composed of multiple elements systematically organized and interacting with each other. The system refers to the overall configuration of hardware, software, an operating system, a network, etc. in the field of computers.

As used herein, the phrase "machine learning" refers to a technique that gives to a computer an ability to learn without explicit programming, that is, a process by which a functional unit improves its own performance by acquiring new knowledge and skills or reconfiguring existing knowledge and skills. Programming a computer to learn from experience reduces much of effort required to program the details, and a way to build a computer program that can automatically improve from experience is discussed in the machine learning field. Along with the algorithm field, data analysis and machine learning are constituent technologies that serve as a basis of intelligent processing, and are usually used in conjunction with other technologies and thus require knowledge in the cooperated field (domain-specific knowledge; for example, in the medicine field). Its range of applications includes roles such as forecasting (collecting data and predicting what will happen), exploration (finding some prominent feature in the collected data), and testing and description (examining relationships among various elements in the data). Machine learning is based on an indicator of an attainment level of a real-world goal and a user of the machine learning must know the real-world goal. It is then necessary to formulate an indicator that will be better when the goal is attained. Machine learning is an inverse problem and an ill-posed problem where it is unclear whether a solution has been solved or not. A behavior of learned rules is probabilistic (probable) rather than definite. It is necessary to devise an operation on the premise that some uncontrollable parts will remain, and the tailor-made method of the present invention can be said to be a solution to this problem. It is also useful for a user of machine learning to successively select data or information to match the real-world goal while checking a performance metric during training and operation.

For example, linear regression, logistic regression, or support vector machines can be used for machine learning, and cross-validation (CV; also referred to as cross-verification or cross-checking) can be used to calculate a discriminant accuracy of each model. After ranking, the number of features is increased one by one, the machine learning (linear regression, logistic regression, support vector machines, etc.) and the cross-validation can be performed to calculate the discriminant accuracy of each model. This allows for selection of a model with the highest accuracy. In the present invention, any machine learning can be used, and linear, logistic, or support vector machines (SVM), etc. can be used as supervised machine learning.

### (Preferred Embodiment)

Although preferred embodiments will be described below, it should be understood that these embodiments are illustrative of the present disclosure and the scope of the present disclosure is not limited to such preferred embodiments. It should also be understood that those skilled in the art can easily make modifications, changes, etc. within the scope of the present disclosure with reference to the following preferred examples. Those skilled in the art may combine any of these embodiments as appropriate.

### (Preservation method)

For cryopreservation of corneal endothelial cells, corneal endothelial cells can be preserved with high viability by freezing in a preservation solution containing 10% DMSO which reduces damage during freezing. However, there were concerns about toxicity of DMSO to cells and irritation when administered to the eye. Therefore, the present inventors have examined preservation conditions that could maintain high viability in a preservation solution with a reduced concentration of DMSO, and have found that a temperature drop at a slower rate than -1°C/min enables maintenance of high viability of corneal endothelial cells in a cryopreservation solution with a reduced concentration (e.g., less than 7%) of or free from DMSO.

If a cooling rate is too slow, water outside the cells is frozen first, which removes the water outside the cells and allows water to flow out of the cells. Cell survival is adversely affected due to an increase in concentration of a solute in the cells. If the cooling rate is too fast, water loss from the cells is reduced, but survival is adversely affected by damage to the cells by ice crystals. The cooling rate upon cryopreservation of the cells has a significant impact on cell damage. An optimal cooling rate can minimize the impact, and a cooling rate of -1°C/min is recommended. It was unexpected that, for corneal endothelial cells, a temperature drop at a slower rate than -1°C/min enabled maintenance of high viability of corneal endothelial cells in a cryopreservation solution with a reduced concentration (e.g., less than 7%) of or free from DMSO.

In one aspect, the present disclosure may provide a method for preserving corneal endothelial cells and/or corneal endothelium-like cells, the method including freezing the corneal endothelial cells and/or the corneal endothelium-like cells in a non-frozen state, the freezing including at least one step of decreasing a temperature at a rate of less than 1°C per minute (a cooling rate slower than -1°C/min) when changing the temperature from a non-frozen temperature to a freezing target temperature.

In one aspect, the present disclosure may provide a method for producing a frozen formulation of corneal endothelial cells and/or corneal endothelium-like cells, the method including freezing the corneal endothelial cells and/or the corneal endothelium-like cells in a non-frozen state optionally mixed with a pharmaceutically acceptable component to thereby produce a frozen formulation, the freezing including at least one step of decreasing a temperature at a rate of less than 1°C per minute when changing the temperature from a non-frozen temperature to a freezing target temperature.

In some embodiments, the cooling rate slower than - 1°C/min may be a temperature in a range of about 0.1°C to about 0.9°C per minute, preferably about 0.2°C to about 0.8°C per minute, and more preferably about 0.2°C to about 0.7°C C per minute. In a certain embodiment, the cooling rate slower than -1°C/min may be -0.9°C/min, -0.8°C/min, -0.7°C/min, -0.6°C/min, -0.5°C/min, -0.4°C/min, -0.3°C/min, -0.2°C/min, or -0.1°C/min.

In one embodiment, the cooling rate to the freezing target temperature (e.g., -80°C) may or may not be constant. In some embodiments, the method of the present disclosure includes at least decreasing a temperature in a specific temperature range at a cooling rate slower than -1°C/min, and may include increasing a temperature, decreasing a temperature at a cooling rate faster than -1°C/min, or maintaining a temperature at a constant temperature in the course of decreasing a temperature to the freezing target temperature.

The target freezing temperature is set as appropriate and may be, for example, a temperature in a range of about -20°C to -196°C such as about -20°C, about -30°C, about -40°C, about - 50°C, about -60°C, about -70°C, about -80°C, about -90°C, about -100°C, about -150°C, about -190°C, or about -196°C.

In one embodiment, the method of the present disclosure may include increasing a temperature, decreasing a temperature at a cooling rate faster than -1°C/min, or maintaining a temperature at a constant temperature as long as a temperature is decreased at an average cooling rate slower than -1°C/min in a specific temperature range.

In one embodiment, the method of the present disclosure may achieve the average cooling rate slower than -1°C/min by decreasing a temperature at a rate faster than -1°C/min in a specific temperature range, maintaining the temperature at a constant temperature, and then decreasing the temperature again at a rate faster than -1°C/min (these procedures may be repeated).

In one embodiment, the method of the present disclosure may achieve the average cooling rate slower than -1°C/min by decreasing a temperature at a rate faster than -1°C/min in a specific temperature range, increasing the temperature, maintaining the temperature at a constant temperature, and then decreasing the temperature again at a rate faster than -1°C/min (these procedures may be repeated).

In one embodiment, the method of the present disclosure may achieve the average cooling rate slower than -1°C/min by decreasing a temperature at a rate faster than -1°C/min in a specific temperature range, increasing the temperature, and then decreasing the temperature again at a rate faster than - 1°C/min (these procedures may be repeated).

In one embodiment, the specific temperature range in which a temperature is decreased at a cooling rate slower than -1°C/min may be a temperature range that includes at least a temperature shifting from at least a non-frozen state to a frozen state. In a certain embodiment, the above-mentioned temperature range may be about -80°C to about 0°C, about -70°C to about 0°C, about -60°C to about 0°C, about -50°C to about 0°C, about -40°C to about 0°C, about -30°C to about 0°C, about -20°C to about 0°C, about -10°C to about 0°C, about -80°C to about -10°C, about -70°C to about -10°C, about -60°C to about - 10°C, about -50°C to about -10°C, about -40°C to about -10°C, about -30°C to about -10°C, or about -20°C to about -10°C.

In one embodiment, the method of the present disclosure may further include maintaining corneal endothelial cells and/or corneal endothelium-like cells in a frozen state. In some embodiments, the freeze maintenance temperature may be about -196°C to about -4°C, about -196°C to about -10°C, about -196°C to about -20 C, about -196°C to about -30°C, about - 196°C to about -40°C, about -196°C to about -50°C, about -196°C to about -60°C, about -196°C to about -70°C, about -196°C to about -80°C, about -80°C to about -4°C, about -80°C to about - 10°C, about -80°C to about -20°C, about -80°C to about -30°C, about -80°C to about -40°C, about -80°C to about -50°C, about - 80°C to about -60°C, or about -80°C to about -70°C. In a preferred embodiment, the freeze maintenance temperature may include maintaining at about -80°C.

In some embodiments, the freezing in the method of the present disclosure may begin at a non-frozen temperature in a range of about 0°C to about 42°C, about 0°C to about 37°C, about 4°C to about 23°C, about 4°C to about 10°C. In a preferred embodiment, especially in the case of freezing in the presence of DMSO, the freezing may begin at a non-frozen temperature of 4°C. In a certain embodiment, the method of the present disclosure may further include incubating corneal endothelial cells and/or corneal endothelium-like cells at the above-mentioned non-frozen temperature prior to the freezing.

In one embodiment, the freezing in the method of the present disclosure may include at least one step of decreasing a temperature at a rate of less than 1°C per minute or maintaining a temperature at a constant temperature for a period of time in at least a portion of or the entirety of a temperature range of about -20°C ± 10°C. In one embodiment, the freezing in the method of the present disclosure may include at least one step of consecutively maintaining a temperature range of about -20°C ± 10°C for a period of time or longer, for example, 20 minutes or longer, 30 minutes or longer, 40 minutes or longer, 50 minutes or longer, 1 hour or longer, 1 hour 30 minutes or longer, or 2 hours or longer. The above-mentioned temperature range may also be -20°C ± 5°C. During the freezing, formation of ice crystals in a non-uniform and unaligned state may increase damage to cells and reduce viability. Without wishing to be bound by any theory, during the freezing, slowly decreasing a temperature near an eutectic point (e.g., -20°C ± 10°C) of the ice crystals and a solute (e.g., NaCl) in a preservation solution or maintaining a temperature near the eutectic point for a period of time causes the crystals to align uniformly and reduce damage to the cells.

A temperature may be varied at any rate outside the temperature range of -20°C ± 10°C as long as at least a step of decreasing a temperature at a rate of less than 1°C per minute or maintaining a temperature at a constant temperature for a period of time in the temperature range of -20°C ± 10°C is included.

In further aspect, the present disclosure may provide a method for preserving corneal endothelial cells and/or corneal endothelium-like cells, the method including freezing the corneal endothelial cells and/or the corneal endothelium-like cells in a non-frozen state, the freezing including at least one step of maintaining a temperature in a temperature range of -20°C ± 10°C for a period of time or longer; and optionally maintaining the corneal endothelial cells and/or the corneal endothelium-like cells in a frozen state. The temperature may be varied at any rate outside the temperature range of -20°C ± 10°C.

In some embodiments, a temperature may be decreased to less than -30°C, increased, and then slowly decreased or maintained for a period of time in a temperature range near an eutectic point (-20°C ± 10°C). Those skilled in the art may adjust a period of time for which a temperature is maintained less than -30°C as appropriate in light of the disclosure herein as long as the effect of the disclosure is achieved, but the period of time may be, for example, 2 hours or less, 1 hour or less, 30 minutes or less, or 20 minutes or less.

In one embodiment, corneal endothelial cells and/or corneal endothelium-like cells can be preserved in a preservation solution containing less than about 7%, about 5% or less, or about 2% or less DMSO. The preservation solution preferably contains about 5% or less, more preferably about 2% or less, and most preferably no DMSO due to a potential adverse effect of DMSO on the cells. In a certain embodiment, the preservation solution may contain about 5% DMSO. In a certain embodiment, the preservation solution may contain about 2% DMSO.

In one embodiment, corneal endothelial cells and/or corneal endothelium-like cells may be frozen in the presence of a ROCK inhibitor.

The ROCK inhibitor may be, for example, compounds disclosed in the following publications: US Patent No. 4678783, Japanese Patent No. 3421217, WO95/28387, WO99/20620, WO99/61403, WO02/076976, WO02/076977, WO2002/083175, WO02/100833, WO03/059913, WO03/062227, WO2004/009555, WO2004/022541, WO2004/108724, WO2005/003101, WO2005/039564, WO2005/034866, WO2005/037197, WO2005/037198, WO2005/035501, WO2005/035503, WO2005/035506, WO2005/080394, WO2005/103050, WO2006/057270, WO2007/026664, WO2014/113620, WO2019/089868, WO2014/055996, WO2019/014300, WO2019/014304, WO2018/138293, WO2018/1115383, WO2018/118109, WO2018/102325, WO2018/009622, WO2018/009625, WO2018/009627, WO2017/205709, WO2017/123860, WO2016/112236, WO2016/028971, WO2015/165341, WO2015/054317, WO2015/002926, WO2015/002915, WO2014/068035, WO2014/055996, WO2013/030366, WO2012/146724, WO2011/107608, WO2010/104851, WO2008/077550, WO2008/036540, WO2005/097790, etc. Such compounds can be produced according to the methods described in the above-disclosed publications. Specific examples thereof include 1-(5-isoquinolinesulfonyl)homopiperazine or salts thereof (e.g., Fasudil (1-(5-isoquinolinesulfonyl)homopiperazine), (+)-trans-4-(1-aminoethyl)-1-(4-pyridylcarbamoyl)cyclohexane ((R)-(+)-trans-(4-pyridyl)-4-(1-aminoethyl)-cyclohexanecarboxamide) or salts thereof (e.g., Y-27632 ((R)-(+)-trans-(4-pyridyl)-4-(1-aminoethyl)-cyclohexanecarboxamide dihydrochloride monohydrate), and the like. Commercially available products of these compounds (from FUJIFILM Wako Pure Chemical Corporation, ASAHI KASEI PHARMA CORPORATION, etc.) can also be suitably used.

In some embodiments, examples of the ROCK inhibitor that may be used include Y-27632 ((+)-trans-4-(1-aminoethyl)-1-(4-pyridylcarbamoyl)cyclohexane, Ripasudil (4-fluoro-5-{[(2S)-2-methyl-1,4-diazepane-1-yl]sulfonyl}isoquinoline), Fasudil (1-(5-isoquinolinesulfonyl)homopiperazine), Verosudil (N-(1,2-dihydro-1-oxo-6-isoquinolinyl)-α-(dimethylamino)-3-thiopheneacetamide), Belumosudil (2-[3-[4-[(1H-indazol-5-yl)amino]quinazolin-2-yl]phenoxy]-N-isopropylacetamide), and pharmaceutically acceptable salts thereof. Belumosudil has the following structure:

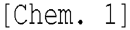

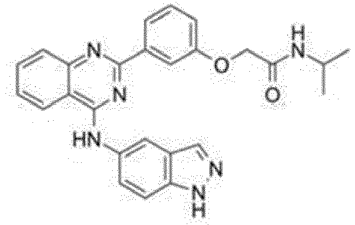

In some embodiments, the ROCK inhibitor may be Ripasudil, Y-27632, Fasudil, Netarsudil, Verosudil, Belumosudil, or pharmaceutically acceptable salts thereof, and more preferably Ripasudil, Y-27632, or pharmaceutically acceptable salts thereof.

### (Formulation)

In another aspect, the present disclosure can provide a frozen formulation of corneal endothelial cells and/or corneal endothelium-like cells produced by the above-mentioned method for preserving corneal endothelial cells and/or corneal endothelium-like cells or by the above-mentioned method for producing a frozen formulation of corneal endothelial cells and/or corneal endothelium-like cells.

In one aspect, the present disclosure can provide a frozen formulation including less than 7% DMSO and corneal endothelial cells and/or corneal endothelium-like cells.

In one aspect, the present disclosure can provide a frozen formulation including less than 7% DMSO and corneal endothelial cells and/or corneal endothelium-like cells in a slow-frozen state.

In one aspect, the present disclosure can provide a frozen formulation including less than 7% DMSO, corneal endothelial cells and/or corneal endothelium-like cells, and a saline component in a frozen state.

In one aspect, the present disclosure can provide a frozen formulation including less than 7% DMSO, corneal endothelial cells and/or corneal endothelium-like cells, and a medium component in a frozen state.

A frozen cell formulation to be used for regenerative medicine includes at least 7% or more DMSO, and therefore, when it is administered to a patient, it was necessary to avoid administering a high concentration of DMSO to the patient by diluting the formulation with saline immediately prior to administration or administering the formulation at an extremely slow rate, for example, in the case of intravenous infusion due to a concern about toxicity of DMSO to the patient. The method of the present disclosure maintained high viability even when preserved in a preservative solution with a reduced DMSO concentration of less than 7%. The present disclosure achieved a frozen formulation containing less than 7% DMSO, which was a previously unachievable low concentration.

In one aspect, a post-thaw long-term stable frozen cell formulation containing less than 7% DMSO and corneal endothelial cells and/or corneal endothelium-like cells can be formulated by the present disclosure.

In one aspect, the present disclosure can provide a frozen formulation including a ROCK inhibitor and corneal endothelial cells and/or corneal endothelium-like cells in a frozen state.

In one aspect, the present disclosure can provide a frozen formulation including a ROCK inhibitor, corneal endothelial cells and/or corneal endothelium-like cells, and a saline component (e.g., NaCl) in a frozen state.

In one aspect, the present disclosure can provide a frozen formulation including a ROCK inhibitor, corneal endothelial cells and/or corneal endothelium-like cells, and a medium component in a frozen state. Those skilled in the art can select the medium component as appropriate. Examples of the medium component include, but are not limited to, a carbon source such as glucose, an amino acid, a vitamin, an electrolyte, phosphate, a buffering agent, a growth factor, serum, and serum albumin. An amino acid included in a component of a basal medium is not particularly limited, and examples thereof include L-arginine, L-cystine, L-glutamine, glycine, L-histidine, L-isoleucine, L-leucine, L-lysine, L-methionine, L-phenylalanine, L-serine, L-threonine, L-tryptophan, L-tyrosine, and L-valine. A vitamin included in a component of a basal medium is not particularly limited, and examples thereof include calcium D-pantothenate, choline chloride, folic acid, i-inositol, niacinamide, riboflavin, thiamine, pyridoxine, biotin, lipoic acid, vitamin B₁₂, adenine, and thymidine. An electrolyte included in a medium component is not particularly limited, and examples thereof include CaCl₂, KCl, MgSO₄, NaCl, NaH₂PO₄, NaHCO₃, Fe(NO₃)₃, FeSO₄, CuSO₄, MnSO₄, Na₂SiO₃, (NH₄)6Mo₇O₂₄, NaVO₃, NiCl₂, and ZnSO₄. When used for cell infusion therapy, a medium is preferably free from a heterologous serum component. As used herein, the phrase "heterologous serum component" means a serum component derived from an organism of a different species from a recipient. For example, if the recipient is human, serum derived from cow or horse, such as fetal bovine serum (FBS), fetal calf serum (FCS), calf serum (CS), or horse serum (HS) comes under the heterologous serum component.

In one aspect, the present disclosure can provide a post-thaw long-term stable frozen cell formulation including corneal endothelial cells and/or corneal endothelium-like cells and a ROCK inhibitor in a slow-frozen state.

In one aspect, the present disclosure can provide a frozen formulation including corneal endothelial cells and/or corneal endothelium-like cells and a ROCK inhibitor both in a slow frozen state, the formulation not inhibiting engraftment and survival in vivo of the corneal endothelial cells and/or the corneal endothelium-like cells administered after thawing.

The formulation of the present disclosure can be administered directly to the eye after thawed.

In one embodiment, the number of corneal endothelial cells and/or corneal endothelium-like cells included in the formulation may be about 1 × 10⁵ to about 3 × 10⁶ cells, preferably about 5 × 10⁵ to about 1 × 10⁶ cells. In a certain embodiment, the number of corneal endothelial cells and/or corneal endothelium-like cells included in the formulation can be, for example, about 1 × 10⁵ cells, about 2 × 10⁵ cells, about 3 × 10⁵ cells, about 4 × 10⁵ cells, about 5 × 10⁵ cells, about 6 × 10⁵ cells, about 7 × 10⁵ cells, about 8 × 10⁵ cells, about 9 × 10⁵ cells, about 1 × 10⁶ cells, about 2 × 10⁶ cells, about 3 × 10⁶ cells, about 4 × 10⁶ cells, or about 5 × 10⁶ cells.

In one embodiment, a liquid volume of the formulation may be about 50 µL to about 2000 µL, about 50 µL to about 1000 µL, about 50 µL to about 800 µL, about 50 µL to about 600 µL, about 50 µL to about 300 µL, about 100 µL to about 2000 µL, preferably about 100 µL to about 1000 µL, more preferably about 200 µL to about 800 µL, and most preferably about 300 µL to about 600 µL, but can be changed as appropriate in accordance with purposes. Product specification may be, for example, ± about 5%, ± about 10%, ± about 15%, ± about 20%, ± about 25%, ± about 50%, etc. of a reference amount (e.g., 300 µL). For example, the liquid volume of the formulation may be at least about 50 µL, e.g., about 100 µL, about 200 µL, about 300 µL, about 400 µL, about 500 µL, about 600 µL, about 700 µL, about 800 µL, about 900 µL, about 1 mL, about 2 mL, about 3 mL, about 4 mL, about 5 mL, about 6 mL, about 7 mL, about 8 mL, about 9 mL, or about 10 mL. In some embodiments, when injection to both eyes is contemplated in cellular infusion therapy, the product specification may be double, triple, or quadruple a dosage. Even if injection to both eyes is not contemplated, the product specification may be double, triple, or quadruple a dosage in case administration fails. The above-mentioned values may be combined into a liquid volume range as appropriate.

In one embodiment, the formulation of the present disclosure may be administered in a volume of about 50 µL to about 350 µL, about 250 µL to about 350 µL, about 300 µL to about 350 µL, or about 300 µL per dose. The formulation of the present disclosure can be administered into the anterior chamber.

A cell density of the formulation of the present disclosure is usually about 2 × 10⁴ cells/mL or more. Without wishing to be bound by any theory, when used for cell injection, an excessively low cell density may not have a therapeutic effect, while an excessively high cell density may promote cell death during preservation due to increased overlapping of cells. Thus, the cell density can typically be determined as appropriate within a range of about 2 × 10⁴ cells/mL to about 8 × 10⁷ cells/mL, preferably about 2 × 10⁴ cells/mL to about 8 × 10⁷ cells/mL, more preferably about 2 × 10⁵ cells/mL to about 8 × 10⁶ cells/mL, further preferably about 1 × 10⁶ cells/mL to about 8 × 10⁶ cells/mL, and most preferably from about 2 × 10⁶ cells/mL to about 4 × 10⁶ cells/mL. Those skilled in the art can determine an adequate cell density in accordance with applications as appropriate. For example, when preserved corneal endothelial cells and/or corneal endothelium-like cells are used for cell infusion therapy, the cell density may be determined so that an operation of adjusting a density after preservation is reduced taking a volume of a suspension to be preserved, an optimal dosage, a ROCK inhibitor to be optionally added, and a volume of a suspension to be administered, etc. into account. Typically, the optimal dosage may be about 1 × 10⁵ to about 3 × 10⁶ cells and preferably about 5 × 10⁵ to about 1 × 10⁶ cells. Those skilled in the art can determine the number of cells contained in the formulation, a liquid volume, and a cell density as appropriate to achieve the optimal dosage.

In one embodiment, corneal endothelial cells and/or corneal endothelium-like cells can be contained in a container. In some embodiments, any container may be used including, but not limited to, a plate (12, 24, 48, or 96-well plate), a tube, a vial (glass vial), a syringe, and a dish. The method of the present disclosure can preserve cells with a high cell viability rate regardless of type of the container.

In one embodiment, the formulation can include less than about 7%, about 5% or less, or about 2% or less DMSO. The formulation preferably includes about 5% or less DMSO, more preferably about 2% or less DMSO, and most preferably no DMSO. In a certain embodiment, the DMSO included in the formulation may be about 5%. In a certain embodiment, the DMSO included in the formulation may be about 2%.

In one embodiment, the formulation can include a ROCK inhibitor. The ROCK inhibitor is as described above. Since the ROCK inhibitor promotes cell adhesion, when the ROCK inhibitor is included in the formulation during preservation, adhesion may be promoted to adversely affect preservation. Therefore, the ROCK inhibitor is typically added to the formulation immediately prior to administration. Unexpectedly, however, when the ROCK inhibitor was previously included in the formulation during preservation, high cell viability was achieved after preservation, and corneal endothelial cells and/or corneal endothelium-like cells injected into the anterior chamber were engrafted and normally functioned in the corneal endothelium (Example 5).

In some embodiments, corneal endothelial cells and/or corneal endothelium-like cells can have viability of at least 80% or at least 90% viability for at least 6 hours at room temperature after thawing. In some embodiments, corneal endothelial cells and/or corneal endothelium-like cells can have viability of at least 90% viability for at least 3 hours at room temperature after thawing.

In another aspect, the present disclosure provides a method for preserving corneal endothelial cells and/or corneal endothelium-like cells, the method including freezing the corneal endothelial cells and/or the corneal endothelium-like cells in a non-frozen state, the freezing including decreasing a temperature at a first rate to a first target temperature and decreasing the temperature at a second rate from the first target temperature to a second target temperature; and optionally maintaining the corneal endothelial cells and/or the corneal endothelium-like cells in a frozen state, the first rate being a rate of less than 1°C per minute and slower than the second rate. The phrase "first target temperature" refers to a temperature at which a supercooled state is maintained. The first target temperature can preferably be a temperature at which freezing begins when cooled at a rate faster than a cooling rate to the first target temperature. The second target temperature refers to a final target temperature achieved by further decreasing a temperature from the first target temperature. The present inventors have found that cell viability is further improved by slowly decreasing a temperature at a first rate in a supercooled state to a first target temperature, changing the first rate to a second rate to initiate freezing, and then decreasing the temperature to a second target temperature. The method may have one or more embodiments described in the present disclosure.

In another aspect, the freezing can include decreasing a temperature at a first rate to a first target temperature and decreasing the temperature at a second rate from the first target temperature to a second target temperature. The first rate is less than 1°C per minute and can be slower than the second rate. The method may have one or more embodiments described in the present disclosure.

In some embodiments, the freezing process may further include decreasing a temperature to a first target temperature and then maintaining the temperature at the first target temperature. A period of time during which the first target temperature is maintained may be set as appropriate as long as a supercooled state is maintained and may be, for example, about 5 minutes or longer, about 10 minutes or longer, about 20 minutes or longer, about 30 minutes or longer, about 40 minutes or longer, about 50 minutes or longer, about 60 minutes or longer, about 70 minutes or longer, about 80 minutes or longer, about 90 minutes or longer, about 100 minutes or longer, about 110 minutes or longer, about 120 minutes or more, about 150 minutes or more, about 180 minutes or more, and up to about 240 minutes.

The first target temperature may be any temperature at which a supercooled state is maintained, for example, a temperature from about -20°C to about -5°C, preferably from about -15°C to about -10°C, and more preferably -13°C to -10°C.

The second target temperature is lower than the first target temperature and can be set as appropriate. For example, it may be a temperature of about -20°C or less, preferably from about -196°C to about -80°C, and more preferably about -196°C or about -80°C.

The first rate may be set as appropriate as long as it is a slow rate. For example, it may be a rate of about 0.9°C or less per minute, preferably about 0.5°C to about 0.05°C per minute, and more preferably about 0.3°C to about 0.1°C per minute.

The second rate may be set as appropriate as long as it is faster than the first rate and is a temperature so that freezing begins when a rate is changed from the first rate to the second rate. For example, it may be a rate of about 0.5 to about 5°C per minute, preferably about 1 to about 3°C per minute.

In one embodiment, corneal endothelial cells and/or corneal endothelium-like cells can be used for cell infusion therapy. In one embodiment, the formulation can be administered after thawing without further processing or culturing.

### (Preservation device)

In further aspect, the present disclosure may provide a device for preserving corneal endothelial cells and/or corneal endothelium-like cells, the method including a housing/preserver that contains a container for containing the corneal endothelial cells and/or the corneal endothelium-like cells; a temperature controller that gives an instruction to control a temperature of the corneal endothelial cells and/or the corneal endothelium-like cells in the container contained in the housing/preserver; and a temperature regulator that is able to regulate the temperature in the housing/preserver based on the instruction from the temperature controller, the temperature controller being able to give an instruction to control the temperature so as to include at least one step of changing the temperature at a rate of less than 1°C per minute when decreasing the temperature from a non-frozen temperature to a freezing target temperature and optionally to maintain the corneal endothelial cells and/or the corneal endothelium-like cells in a frozen state.

In another aspect, the temperature controller in the device of the present disclosure may give an instruction to decrease a temperature at a first rate to a first target temperature and then decrease the temperature at a second rate from the first target temperature to a second target temperature when decreasing the temperature from a non-frozen temperature to a freezing target temperature. The device may have one or more embodiments described in the present disclosure.

In a further aspect, the present disclosure may provide a program for causing a computer to execute a method for preserving corneal endothelial cells and/or corneal endothelium-like cells in a device, the device including a housing/preserver that contains a container for containing the corneal endothelial cells and/or the corneal endothelium-like cells; a temperature controller that gives an instruction to control a temperature of the corneal endothelial cells and/or the corneal endothelium-like cells in the container contained in the housing/preserver; and a temperature regulator that is able to regulate the temperature in the housing/preserver based on the instruction from the temperature controller, the program causing the temperature controller to execute controlling the temperature so as to include at least one step of changing the temperature at a rate of less than 1°C per minute when decreasing the temperature from a non-frozen temperature to a freezing target temperature; and optionally maintaining the corneal endothelial cells and/or the corneal endothelium-like cells in a frozen state.

In another aspect, the program of the present disclosure may give an instruction to decrease a temperature at a first rate to a first target temperature and then decrease the temperature at a second rate from the first target temperature to a second target temperature when decreasing the temperature from a non-frozen temperature to a freezing target temperature. The program may have one or more embodiments described in the present disclosure.

In a further aspect, the present disclosure may provide a recording medium that stores a program for causing a computer to execute a method for preserving corneal endothelial cells and/or corneal endothelium-like cells in a device, the device including a housing/preserver that contains a container for containing the corneal endothelial cells and/or the corneal endothelium-like cells; a temperature controller that gives an instruction to control a temperature of the corneal endothelial cells and/or the corneal endothelium-like cells in the container contained in the housing/preserver; and a temperature regulator that is able to regulate the temperature in the housing/preserver based on the instruction from the temperature controller, the program causing the temperature controller to execute controlling the temperature so as to include at least one step of changing the temperature at a rate of less than 1°C per minute when decreasing the temperature from a non-frozen temperature to a freezing target temperature; and optionally maintaining the corneal endothelial cells and/or the corneal endothelium-like cells in a frozen state.

In another aspect, the program stored in the recording medium of the present disclosure may give an instruction to decrease a temperature at a first rate to a first target temperature and then decrease the temperature at a second rate from the first target temperature to a second target temperature when decreasing the temperature from a non-frozen temperature to a freezing target temperature. The program may have one or more embodiments described in the present disclosure.

Various functions realized by the device or the program of the present disclosure may be realized in part or in whole manually.

The various functions realized by the device or the program of the present disclosure may be realized or optimized in part or in whole by artificial intelligence (AI) or machine learning.

The program according to the present disclosure may be stored in a computer-readable recording medium or may be configured as a program product. As used herein, the phrase "recording medium" shall include any "portable physical medium" such as a memory card, a USB memory, an SD card, a flexible disk, a magneto-optical disk, a ROM, an EPROM, an EEPROM, a CD-ROM, a MO, a DVD, and a Blue-ray (registered trademark) Disc.

The term "program" is a data processing method written in any language or description method, and may be in any format such as source code or binary code. Note that, the "program" is not necessarily limited to those that are composed singly, but includes those that are distributed as multiple modules or libraries and those that achieve their functions in cooperation with a separate program represented by an operating system (OS). Well-known configurations and procedures can be used for a specific configuration for reading a recording medium in each device described in the embodiments, a reading procedure, or an installation procedure after reading.

Various databases are storage means, for example, memory devices such as RAM and ROM; fixed disk devices such as hard disks; flexible disks, or optical disks, and store various programs, tables, databases, web page files, or the like to be used for various processing and website provision.

Furthermore, a specific form of distribution and integration of devices is not limited to those shown in figures, but can be composed in whole or in part by functionally or physically distributing and integrating them in arbitrary units in accordance with various additions or in accordance with functional loads. In other words, any combination of the above-mentioned embodiments may be implemented, or the embodiments may be implemented selectively.

### (Kit)

In a further aspect, the present disclosure may provide a frozen formulation kit including a container that contains a frozen formulation including a ROCK inhibitor and corneal endothelial cells and/or corneal endothelium-like cells both in a frozen state; and a housing that contains the container while maintaining in a frozen state.

In one aspect, the present disclosure may provide a frozen formulation kit including a container that contains a frozen formulation including corneal endothelial cells and/or corneal endothelium-like cells in a frozen state; and a housing that contains the container while maintaining in a frozen state.

In one aspect, the present disclosure may provide a frozen formulation kit including a formulation according to the present disclosure; a container that contains the formulation; and a housing that contains the container while maintaining the formulation in a frozen state.

In one aspect, the present disclosure may provide a frozen formulation kit including a container; and a housing that contains the container, the container being used to contain the formulation according to the present disclosure, and the housing being used to maintain the formulation in a frozen state.

In one aspect, the present disclosure may provide a use of a kit, the kit including a container; and a housing that contains the container, the container being used to contain the formulation according to the present disclosure, and the housing being used to maintain the formulation in a frozen state.

In one embodiment, the housing can maintain a container to be contained at a temperature in a range of about -80°C to about -20°C. In some embodiments, the housing can maintain a container to be contained at about -80°C.

### (Preservation, transportation, and treatment)

In a further aspect, the present disclosure may provide a method for transporting and/or preserving a formulation according to the present disclosure, including placing the formulation in a container of a kit including the container and a housing that contains the container, and maintaining the formulation in the kit in a frozen state.

In further aspect, provided may be a method for performing a corneal endothelial cell injection therapy, the method including providing corneal endothelial cells and/or corneal endothelium-like cells suitable for the cell infusion therapy; freezing the corneal endothelial cells and/or the corneal endothelium-like cells, the freezing including at least one step of decreasing a temperature of the corneal endothelial cells and/or the corneal endothelium-like cells at a rate of less than 1°C per minute from a non-frozen temperature; maintaining the corneal endothelial cells and/or the corneal endothelium-like cells in a frozen state and optionally transporting them to the infusion therapy; thawing the corneal endothelial cells and/or the corneal endothelium-like cells; and administering the corneal endothelial cells and/or the corneal endothelium-like cells to a subject.

In another aspect, the method of the present disclosure may include, when decreasing a temperature from a non-frozen temperature to a freezing target temperature, decreasing a temperature at a first rate to a first target temperature and decreasing the temperature at a second rate from the first target temperature to a second target temperature. The method may have one or more embodiments described in the present disclosure.

In one embodiment, transportation can be performed while maintaining a temperature in a range of about -80°C to about - 20°C, preferably -80°C.

Administration to a subject is preferably performed within 6 hours of thawing, for example, the administration may be performed within 6 hours, within 5 hours, within 4 hours, within 3 hours, within 2 hours, within 1 hour, within 30 minutes, within 20 minutes, or within 10 minutes. Administration to a subject can be performed in the anterior chamber of the eye.

The present disclosure has been described above with preferred embodiments for ease of understanding. Hereinafter, the present disclosure will be described with reference to Examples, but the above description and the following examples are provided for illustrative purposes only and are not intended to limit the present disclosure. Accordingly, the scope of the present disclosure is not limited to embodiments or examples specifically described herein, but is limited only by the claims.

### [Example]

The present disclosure will be specifically described with reference to Examples. It is understood that the various reagents used in Examples can be obtained from Sigma-Aldrich, BASF Japan K.K., and the like, in addition to those specifically indicated.

### (Example 1: Freezing in cryopreservation solution containing known components)

Glycerin and polyethylene glycol are well-known components as a cell cryopreservative, but these components alone are not effective in preserving cells. Therefore, a protein component such as albumin or 10% DMSO has been commonly added. A purpose of this example is to verify whether HCECs can be preserved under such general conditions and whether HCECs can be cryopreserved even with a lower concentration of DMSO when a cooling rate is slower than -1°C/min, the commonly known rate.

### (Material)

· Human corneal endothelial cells after 4 passages
· OptiMEM^{™}-I (Invitrogen 21585-070)
· Tryple^{™} Select Enzyme (10x) (Thermo Fisher Scientific A12177-01)
· Sekijuji Albumin 25% I.V. 12.5 g/50 mL (Japan Blood Products Organization)
· 2.0 mL Cryogenic Vials (Corning 430488)
· Programmable freezer (NEPAGENE PF-NP-200)
· Bicell (Nihon Freezer Co., Ltd.)
· iMatrix-511 (nippi 892012)
· Bambanker hRM (NIPPON Genetics CS-11-001)
· Cryostor CS10 preformulated with 10% DMSO (Hemacare 210102)
· Cryostor CS5 preformulated with 5% DMSO (Hemacare 205102)
· Cryostor CS2 preformulated with 2% DMSO (Hemacare 202102)
· CP-1 high grade (Kyokuto Pharmaceutical 27207)
· Bambanker DMSO Free (NIPPON Genetics CS-09-001)
· CryoScarless DMSO Free (BioVerde CPL-A1)
· 12-well plate (costar 3513)
· PROTEOSAVE SS 50 mL centrifuge tube (Sumitomo Bakelite MS-52550)
· STEMFULL 15 mL centrifuge tube (Sumitomo Bakelite MS-90150)

OptiMEM containing 4% HSA, 10% glycerin or 10% polyethylene glycol, and 10%, 5%, 2%, or 0% DMSO was used as a preservation solution.

### (Method)

1. Use Human corneal endothelial cells after 4 passages. Remove a medium from a culture dish in culture, add OptiMEM, and wash. Repeat these procedures twice.
2. After removal of OptiMEM, add Tryple^{™} Select Enzyme (10x) and incubate at 37°C (5% CO₂) for 20 minutes.
3. Twenty minutes later, suspend in a medium, and collect in a 50 mL STEMFULL.
4. Centrifuge at 300G for 5 minutes.
5. Remove a supernatant and suspend in OptiMEM (2% HSA).
6. Centrifuge at 300G for 5 minutes.
7. Repeat Steps 5 and 6 again.
8. Remove a supernatant, suspend in OptiMEM (2% HSA), and count the number of cells by trypan blue staining.
9. Dispense the cells into 15 mL STEMFULLs, the number of which corresponds to that of cryopreservation reagents to be tested, so that each contains 1.2 × 10⁶ cells. Seed cells in a 12-well plate at a cell density of 1000 cells/mm² as an unfrozen group (control) and culture for 2 weeks changing a medium every 2 days.
10. Centrifuge the 15 mL STEMFULLs at 300 G for 5 minutes.
11. Collect and preserve cells in cryotubes each containing a cryopreservation solution.
12. Freeze in a programable freezer from 4°C to -80°C at - 0.5°C/min.
13. Transfer to a Bicell processing housing previously cooled at -80°C when a temperature was decreased to -80°C and cryopreserve the Bicell in a freezer at -80°C for 3 days.
14. After 3 days of cryopreservation, thaw the cryotubes in which the cells were preserved in a water bath at 37°C for 1 to 2 minutes.
15. Collect the cells in a medium warmed to 37°C in advance, and measure the number of cells collected and cell viability by trypan blue staining.

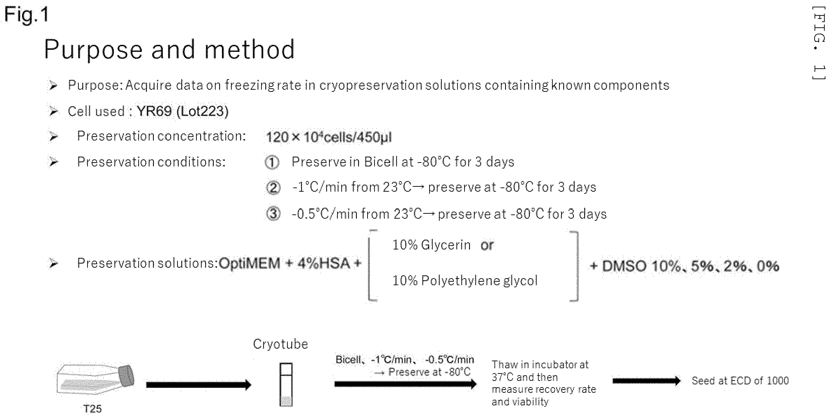

An overview of Example 1 is shown in Figure 1.

### (Results)

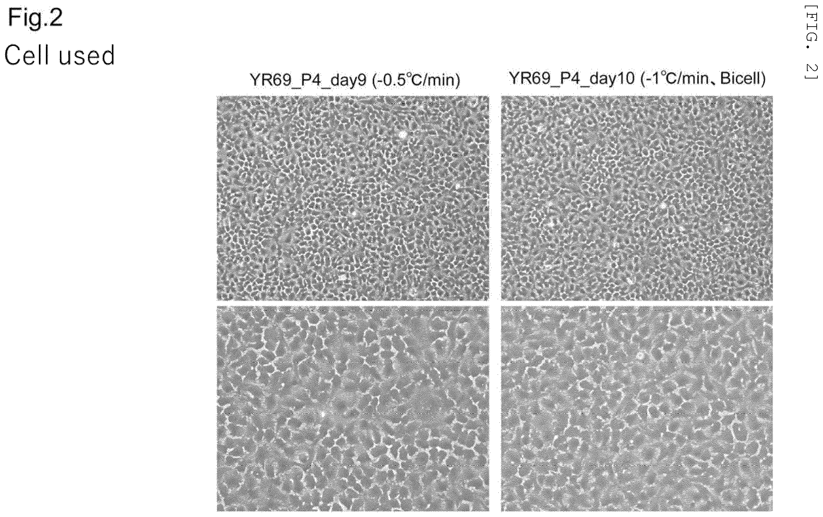

Figure 2 shows photographs of morphology of cultured cells used in this Example. The cells in this lot were found to be free of morphological abnormality.

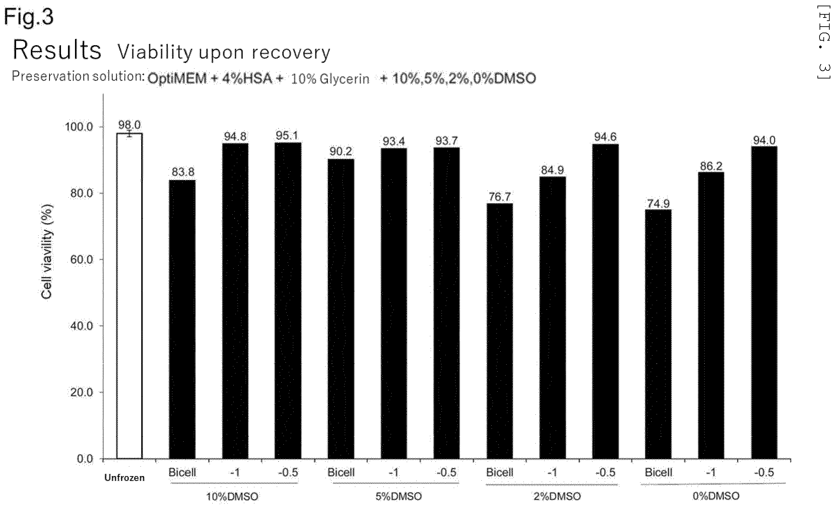

Figure 3 shows a graph comparing cell viability after being preserved in cryopreservation solutions with 4% human serum albumin and 10% glycerin serving as base components and different concentrations of DMSO and thawed. When 5% or more DMSO was contained, high viability was maintained regardless of cooling rate. However, at 2% or 0% DMSO, a cooling rate had a significant effect on cell viability and 90% or higher viability was maintained in the case of -0.5°C/min. A Bicell is a housing that contains tubes for cells to be frozen and of which internal temperature decreases at a rate of around - 1°C/min when placed in a deep freezer at -80°C. Cell viability was lower when preserved in the Bicell compared to when preserved in a programmable freezer at -1°C/min.

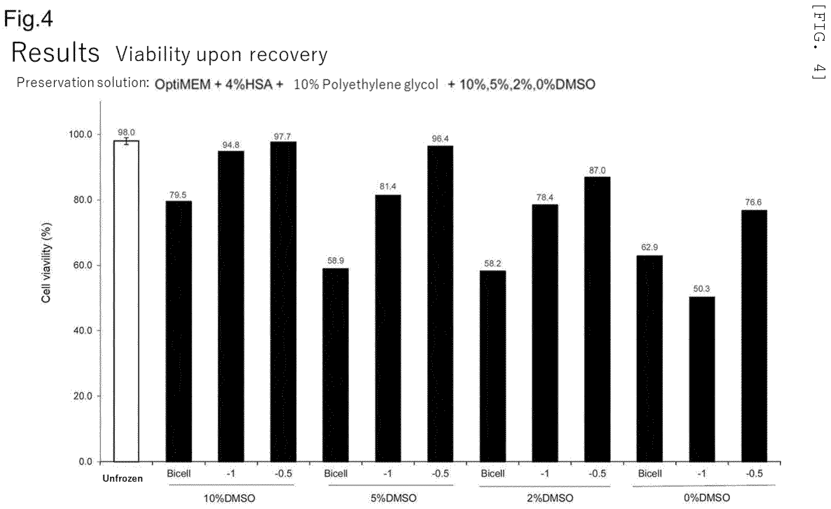

Figure 4 shows a graph comparing cell viability after being preserved in cryopreservation solutions with 4% human serum albumin and 10% polyethylene glycol serving as base components and different concentrations of DMSO and thawed. Unlike the results for the cryopreservation solutions containing glycerin, cell viability in the cryopreservation solutions containing 5% DMSO was also affected by a cooling rate. For compositions containing 2% or less DMSO, when frozen at a cooling rate of -0.5°C/min, viability was about 80% which was much higher than when frozen at a cooling rate of -1°C/min. These results indicate that a slower cooling rate improves viability.

### (Example 2: Examination of cryopreservative and freezing rate) (Material)

· Human corneal endothelial cells after 4 passages
· OptiMEM^{™}-I (Invitrogen 21585-070)
· Tryple^{™} Select Enzyme (10x) (Thermo Fisher Scientific A12177-01)
· Sekijuji Albumin 25% I.V. 12.5 g/50 mL (Japan Blood Products Organization)
· 2.0 mL Cryogenic Vials (Corning 430488)
· Programmable freezer (NEPAGENE PF-NP-200)
· Bicell (Nihon Freezer Co., Ltd.)
· iMatrix-511 (nippi 892012) · Bambanker hRM (NIPPON Genetics CS-11-001)
· Cryostor CS10 preformulated with 10% DMSO (Hemacare 210102)
· Cryostor CS5 preformulated with 5% DMSO (Hemacare 205102)
· Cryostor CS2 preformulated with 2% DMSO (Hemacare 202102)
· CP-1 high grade (KYOKUTO PHARMACEUTICAL 27207)(containing 10% DMSO) Bambanker DMSO Free (NIPPON Genetics CS-09-001)
· CryoScarless DMSO Free (BioVerde CPL-A1)
· 12-well plate (costar 3513)
· PROTEOSAVE SS 50 mL centrifuge tube (Sumitomo Bakelite MS-52550)
· STEMFULL 15 mL centrifuge tube (Sumitomo Bakelite MS-90150)

### (Method)

1. Use Human corneal endothelial cells after 4 passages. Remove a medium from a culture dish in culture, add OptiMEM, and wash. Repeat these procedures twice.
2. After removal of OptiMEM, add Tryple^{™} Select Enzyme (10x) and incubate at 37°C (5% CO₂) for 20 minutes.
3. Twenty minutes later, suspend in a medium and collect in a 50 mL STEMFULL.
4. Centrifuge at 300G for 5 minutes.
5. Remove a supernatant and suspend in OptiMEM (2% HSA).
6. Centrifuge at 300G for 5 minutes.
7. Repeat Steps 5 and 6 again.
8. Remove a supernatant, suspend in OptiMEM (2% HSA), and count the number of cells by trypan blue staining.
9. Dispense the cells into 15 mL STEMFULLs, the number of which corresponds to that of cryopreservation reagents to be tested, so that each contains 1.2 x 10⁶ cells. Seed cells in a 12-well plate at a cell density of 1000 cells/mm² as an unfrozen group (control) and culture for 2 weeks changing a medium every 2 days.
10. Centrifuge the 15 mL STEMFULLs at 300 G for 5 minutes.
11. Suspend in 350 µL of various cryopreservation reagents.
12. Add Y-27632 (100 µL) adjusted to a final concentration of 100 µM to eleven 15 mL STEMFULLs containing the various cryopreservation reagents to make a total volume of 450 µL.
13. Dispense 450 µL of the cells into each cryotube (1.2 x 10⁶ cells/450µL). 14. Freeze in a programable freezer from 4°C to -80°C at -1°C/min or -0.5°C/min, or -0.2°C/min.
15. Transfer to a Bicell processing housing previously cooled at -80°C when a temperature was decreased to -80°C and cryopreserve the Bicell in a freezer at -80°C for 3 days.
16. After 3 days of cryopreservation, thaw the cryotubes in which the cells were preserved in a water bath at 37°C for 1 to 2 minutes.
17. Collect the cells in a medium warmed to 37°C in advance, and measure the number of cells collected and cell viability by trypan blue staining.
18. Collect and reseed the cells in a 12-well plate at a cell density of 1000 cells/mm².
19. Culture for 2 weeks changing the medium every 2 days.
20. On day 7 of culture, photograph the cells on 5 fields under a phase contrast microscope (x200), and calculate a cell density.

### (Results)

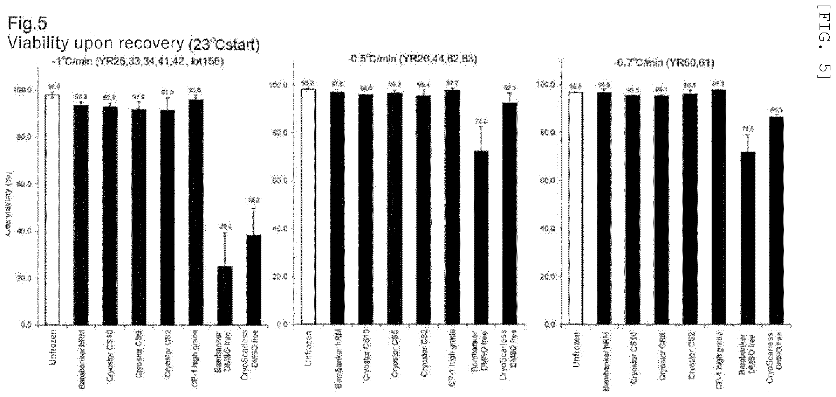

Figure 5 shows data comparing viability after thawing cells frozen at a cooling rate of -1°C/min, -0.5°C/min, or - 0.2°C/min from 4°C. High viability was achieved with the cryopreservatives containing DMSO at -1°C/min, -0.5°C/min, and -0.2°C/min. Cell viability increased in inverse proportion to a cooling rate for the cryopreservatives without DMSO, suggesting that a slower cooling rate is effective in increasing cell viability. When the cells frozen at a cooling rate of -1°C/min were thawed and seeded into an incubator, many non-adherent cells were observed on a bottom surface, indicating a decreased function of corneal endothelial cells. These findings suggest that freezing at a cooling rate slower than -1°C/min is important for improving viability and maintaining a function of corneal endothelial cells.

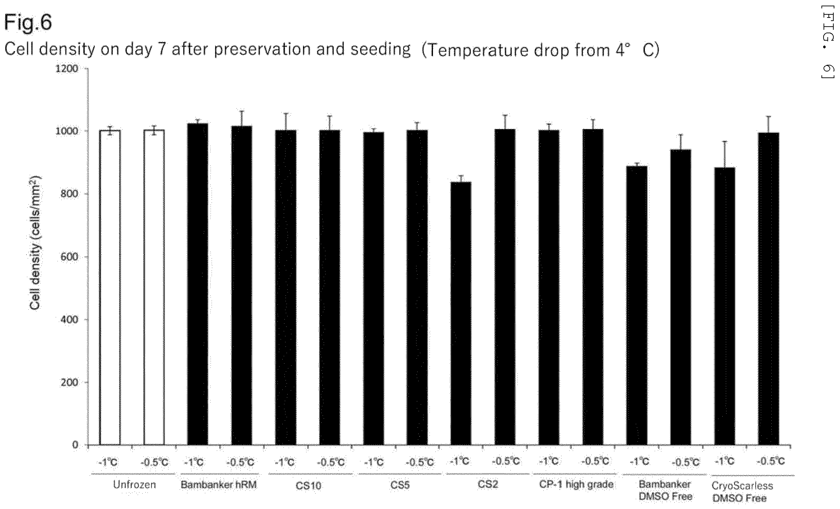

Figure 6 shows data comparing cell densities on day 7 after freezing cells at a cooling rate of -1°C/min or - 0.5°C/min from 4°C, preserving, and then seeding. When frozen at -1°C per minute in CS2 with 2% DMSO, as well as Bambanker DMSO Free and CryoScarless DMSO Free, both of which were free from DMSO, a cell density was low. However, when frozen at a cooling rate of -0.5°C per minute, a high cell density was observed. This indicates that a cooling rate also affects a cell density in culture after preservation.

### (Example 3: Examination of culture with addition of DMSO) (Method)

1. Collect cells in the same manner as in Example 2 (Steps 1 to 10) and preserve the cells in cryotubes using Cryostor CS2.
2. Freeze in a programable freezer from 4°C to -80°C at - 0.5°C/min.
3. Transfer to a Bicell processing housing previously cooled at -80°C when a temperature was decreased to -80°C and cryopreserve the Bicell in a freezer at -80°C for 3 days.
4. After 3 days of cryopreservation, thaw the cryotubes in which the cells were preserved in a water bath at 37°C for 1 to 2 minutes.
5. Collect the cells in a medium warmed to 37°C in advance, and measure the number of cells collected and cell viability by trypan blue staining.
6. Dispense the collected cells into four 15 mL STEMFULLs and centrifuge at 300 G for 5 minutes. 7.After centrifugation, remove a supernatant, adjust a cell density to 1000 cells/mm² using media containing 10%, 5%, 2%, and 0% DMSO, and reseed in 12-well plates. 8. Photograph the cells under a phase contrast microscope at 1 h, 3 h, 6 h, and 24 h after seeding.
9. After taking photographs of the cells at each time point, collect all cells in the container, including floating cells, and determine cell viability by trypan blue staining.

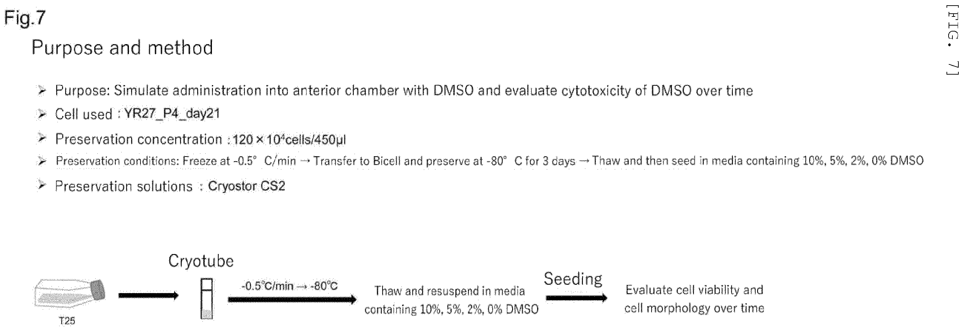

An overview of Example 3 is shown in Figure 7.

### (Results)

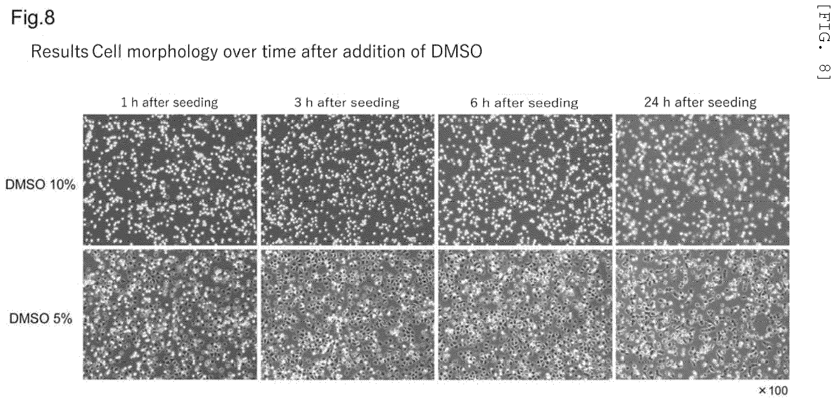

Figure 8 shows phase contrast micrographs of cells cultured in a medium supplemented with 10% or 5% DMSO. The number of cells cultured in the medium supplemented with 10% or 5% DMSO was significantly lower than that of cells cultured in a medium without DMSO, indicating the presence of many non-adherent cells on the (laminin-coated) bottom of an incubator. The majority of the cells cultured in the medium without DMSO were adherent even at 1 hour after inoculation and all cells were adherent at 3 hours, whereas no cells were adherent until 24 hours at 10% DMSO. For 5% DMSO, almost no adherent cells were observed after 1 hour and less than half of the cells were adherent after 3 and 6 hours, but conversely, there were almost no adherent cells after 24 hours. In the micrographs in Figure 8, cells that appear white represent non-adherent cells, and noncircular cells that appear black represent adherent cells.

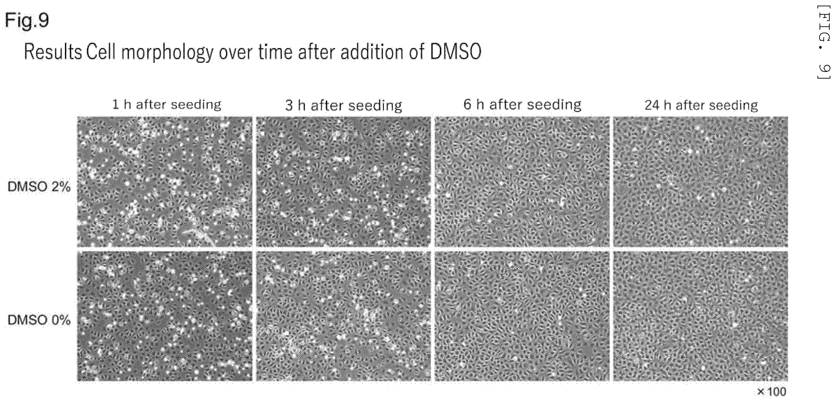

Figure 9 shows phase contrast micrographs of cells cultured in a medium with 2% or without DMSO. An adhesion rate of the cells cultured in the medium containing 2% DMSO was not different from that of the cells cultured in the medium without DMSO.

When cells with a composition containing DMSO are injected into the environment within the anterior chamber of the eye, although the anterior chamber fluid will be gradually replaced, the anterior chamber fluid is believed to be exposed to a high concentration of DMSO for a considerable period of time, and therefore, when cells that are in a cryopreservative with a high concentration of DMSO, such as 10% are injected into the anterior chamber, viability and adhesion of corneal endothelial cells are expected to be severely impaired.

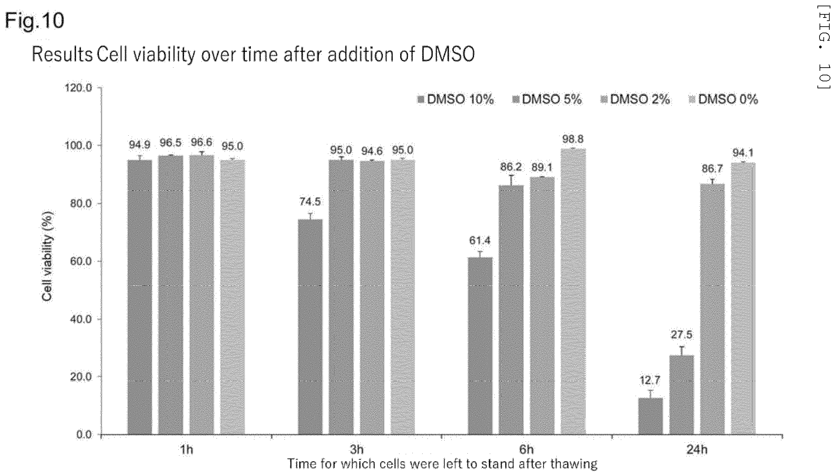

Figure 10 shows a graph showing results of viability of cells after reseeded and collected. The cells cultured in the medium containing 10% DMSO, which were hardly adherent in the micrographs (Figure 8), showed no decrease in viability at 1 hour. However, the subsequent decrease in viability suggests that the cells cultured in the medium containing 10% DMSO had already suffered significant damage at 1 hour although no decrease in viability was observed and were therefore unable to adhere.

Based on these results, a cell formulation to be injected into the anterior chamber preferably includes 5% or less DMSO, and most preferably 2% or less or no DMSO.

### (Example 4: Examination of stability after freezing and thawing)

### (Method)

1. Collect cells in the same manner as in Example 2 (Steps 1 to 10) and preserve the cells in six cryotubes for each preservation solution using Cryostor CS10, CS5, and CS2.
2. Freeze in a programable freezer from 4°C to -80°C at - 0.5°C/min.
3. Transfer to a Bicell processing housing previously cooled at -80°C when a temperature was decreased to -80°C and cryopreserve the Bicell in a freezer at -80°C for 3 days.
4. After 3 days of cryopreservation, thaw the cryotubes in which the cells were preserved in a water bath at 37°C for 1 to 2 minutes.
5. For one for each preservation solution, immediately collect the cells in a medium warmed to 37°C in advance, and measure for cell viability by trypan blue staining (0h). For the remaining five for each preservation solution, leave the cells to stand at room temperature for 30 min, 1 h, 3 h, 6 h, and 24 h, and then collect in the same manner and measure for cell viability by trypan blue staining.
6. Reseed the cells collected at each time point in 12-well plates at a cell density of 1000 cells/mm².
7. Photograph the cells under a phase contrast microscope after 24 hours of culture from reseeding.

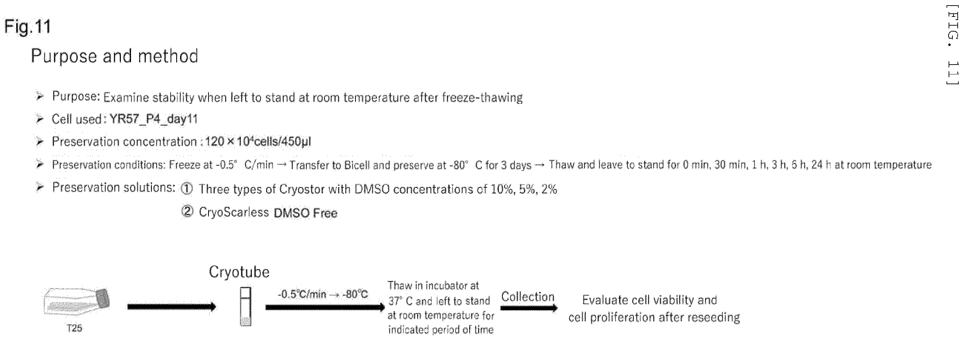

An overview of Example 4 is shown in Figure 11.

### (Results)

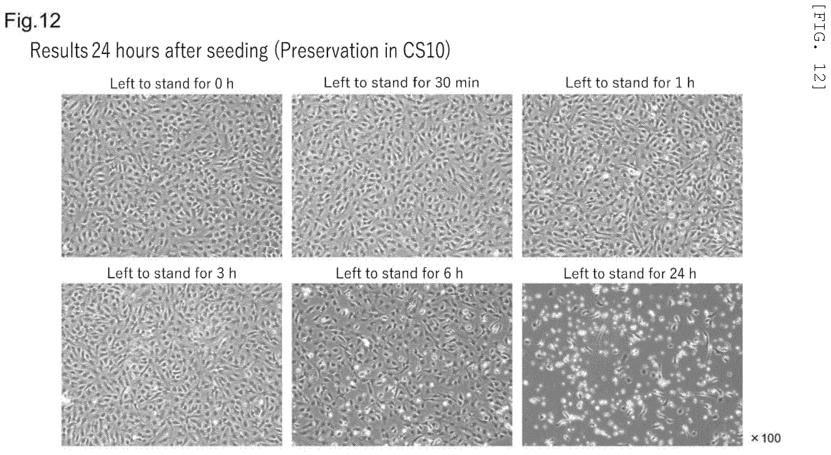

Figure 12 shows phase contrast microscopy images of cells frozen in Cryostor CS10 containing 10% DMSO, left to stand at room temperature for 0 h, 30 min, 1 h, 3 h, 6 h, or 24 h, then reseeded into T25 culture flasks, and 24 hours later photographed in culture. When left to stand at room temperature for 6 hours or longer, a decrease in cell proliferation and adhesive capacity was observed.

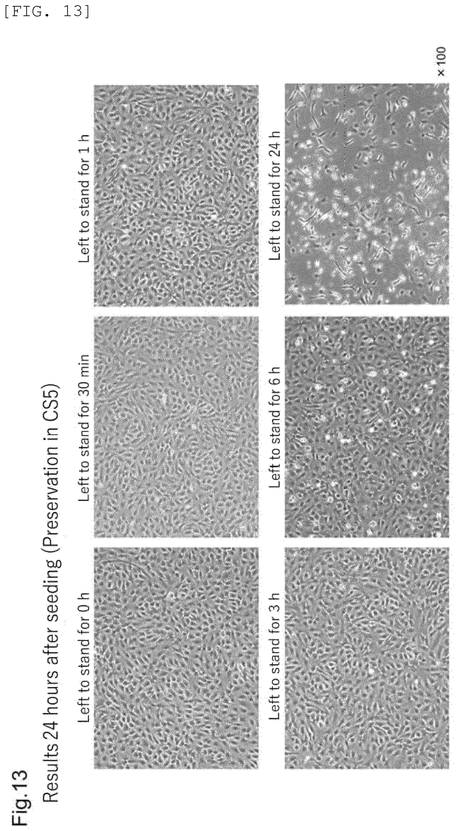

Figure 13 shows phase contrast microscopy images of cells frozen in Cryostor CS5 containing 5% DMSO, left to stand at room temperature for 0 h, 30 min, 1 h, 3 h, 6 h, or 24 h, then reseeded into T25 culture flasks, and 24 hours later photographed in culture. Although a slight decrease in cell proliferation and adhesive capacity was observed in the cells left to stand at room temperature for 6 hours, the degree of decrease was lower than that of CS5.

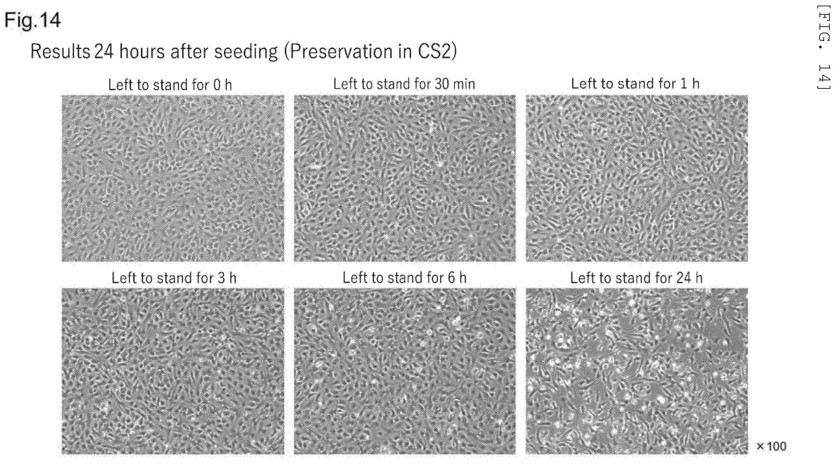

Figure 14 shows phase contrast microscopy images of cells frozen in Cryostor CS2 containing 2% DMSO, left to stand at room temperature for 0 h, 30 min, 1 h, 3 h, 6 h, or 24 h, then reseeded into T25 culture flasks, and 24 hours later photographed in culture. No change is observed even in the cells left to stand at room temperature for 6 hours.

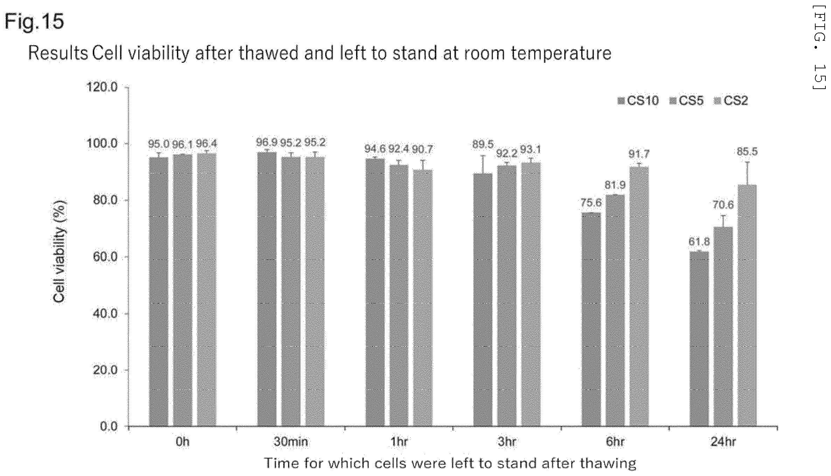

Figure 15 shows a graph showing results of viability of cells after reseeding and collected. These results suggest that cell formulations frozen in cryopreservatives containing 5% or less, preferably 2% or less DMSO are stable even after thawing and are highly convenient for clinical use.

### (Example 5: Cryopreservation in VIXELL^{™} and injection of corneal endothelial cells after preservation)

VIXELL^{™} can maintain -75°C ± 15°C for 18 days by filling with dry ice. In this example, VIXELL^{™} is used to preserve and transport corneal endothelial cells, followed by injection of the corneal endothelial cells.

### (Method)

### (Cryopreservation)

1. Collect cultured corneal endothelial cells and suspend the cells in CryoStor (registered trademark) CS2 supplemented with 100 µM Y27632 at a cell density of 1.2 × 10⁶ cells/450 µL.
2. Freeze vials using a programmable freezer by decreasing a temperature from 4°C to -80°C at a rate of -0.5°C/min.
3. Place the preserved vials in Bicell^{™} and preserve once in a deep freezer at -80°C.
4. Remove the preserved vials and preserve them in VIXELL^{™} containing dry ice for 5 days.

### (Cell injection)

The dynamics of corneal endothelial regeneration was observed in vivo by injecting cultured human corneal endothelial cells cryopreserved in Cryostor CS2 and preserved for 5 days in a cold box for transportation of medical supplies (VIXELL^{™}) to an animal model with corneal endothelial cells detached in an area with a diameter of 8 mm, without perfusion of the anterior chamber fluid.

**[Table 1]**

| | Observation of tissue | ① Endothelium detachment | ② Perfusion | ③ Cell injection |
|---|---|---|---|---|
| (1R-3R: n = 3) | 1 day after injection | Diameter: 8 mm Corneal endothelium detachment | No | Cyrostor CS2 + 8.0 × 10⁵ HCEs + Y-27632 |
| (4R-6R: n = 3) | 5 days after injection | Diameter: 8 mm Corneal endothelium detachment | No | Cyrostor CS2 + 8.0 × 10⁵ HCEs + Y-27632 |

After completion of the 3-hour prone posture, the anterior eye was observed by slit-lamp microscopy at 1, 2, 3, and 5 days to check for inflammation and infection. On the day before, the day of, and 2 and 4 days after surgery, 5 mg/mL of Prograf injection solution was diluted with 100 mL of saline, and a total volume of 6 ml was injected into the posterior auricular vein. Euthanasia and immunostaining were performed on days 1 and 5 after surgery.

### (Results)

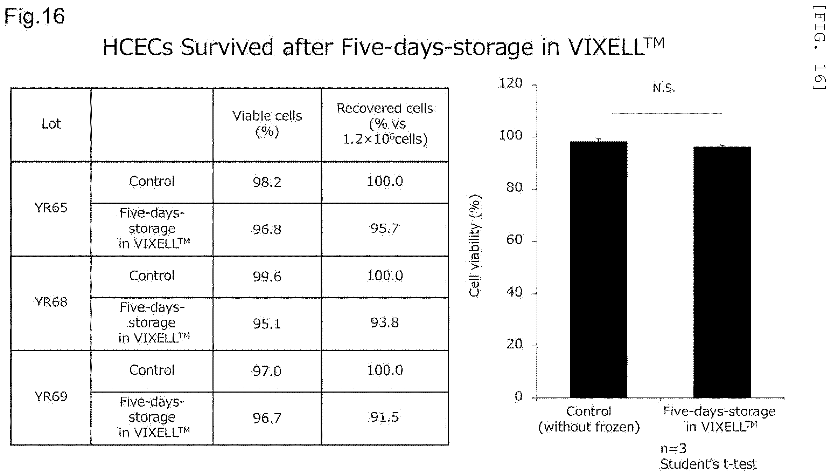

Figure 16 shows viability and cell recovery rate of cells after being preserved in a cold box for 5 days and thawed. The viability was calculated as the number of viable cells/the number of total cells on day 5. The cell recovery rate was defined as a recovery rate taking the theoretical cell count (the number of cells filled) as 100%.

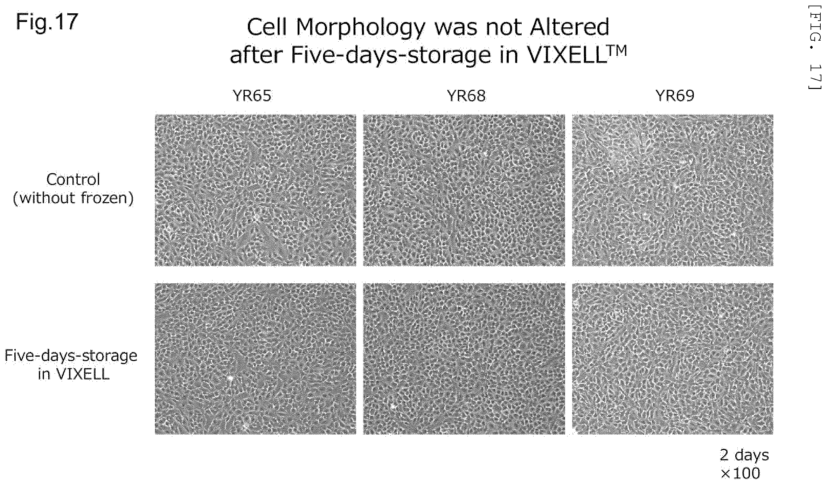

To verify whether the cells preserved in the cold box retained normal morphology, the cells were thawed, removed from cryopreservative by centrifugation, resuspended in an OPTI-MEM medium (also containing Y27632) containing 8% FBS as usual, seeded into T25 culture flasks, and cultured for 2 days. Figure 17 shows micrographs of cells cultured for 2 days after being preserved in a cold box. The cells adhered to the bottom of an incubator in the same shape as non-frozen cells, confirming that normal morphology was retained.

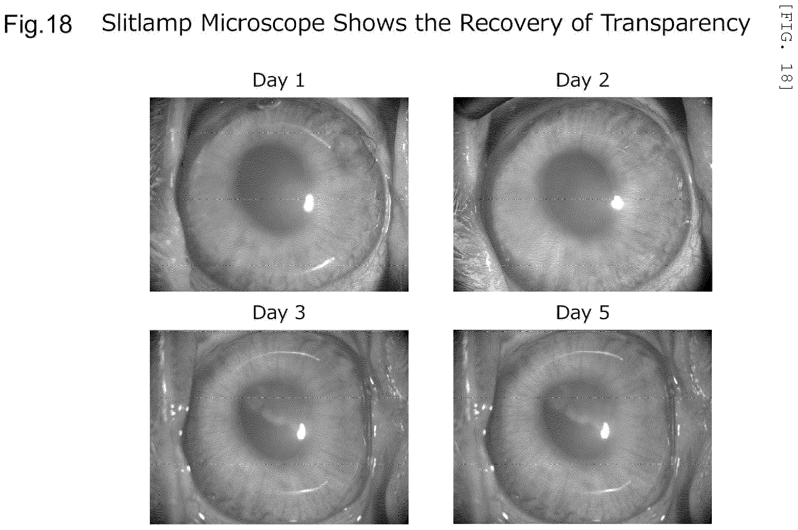

Figure 18 shows photographs of a rabbit eye injected with cells after being preserved in a cold box. Corneal transparency was maintained due to engraftment of corneal endothelial cells.

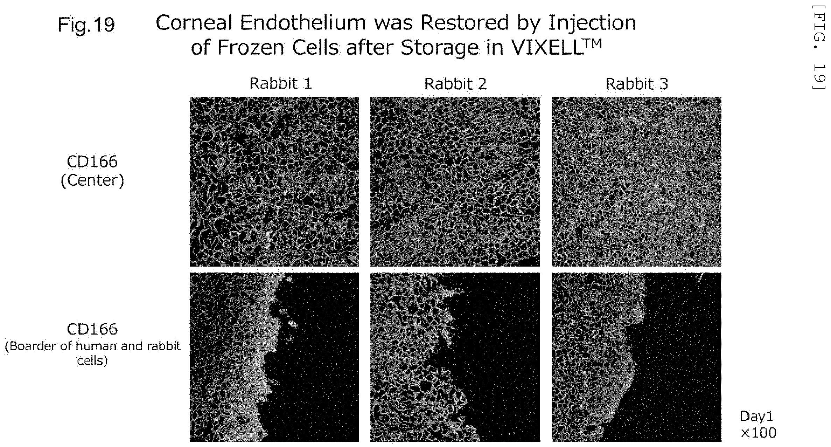

Figure 19 shows photographs of immunohistochemical staining for CD166 in the corneal endothelium on day 1 after cell injection. A corneal endothelial tissue was fixed and immunohistochemically stained by binding to an antibody against CD166, one of expression markers of corneal endothelial cells, as a primary antibody followed by a fluorescently labeled secondary antibody. The injected cells were neatly engrafted as a monolayer, confirming strong expression of CD166. The upper row shows a central part of the cornea and the lower row shows a peripheral part. Because staining was performed with the antibody that binds only to human CD166, rabbit corneal endothelium was not stained and a clear border was identified.

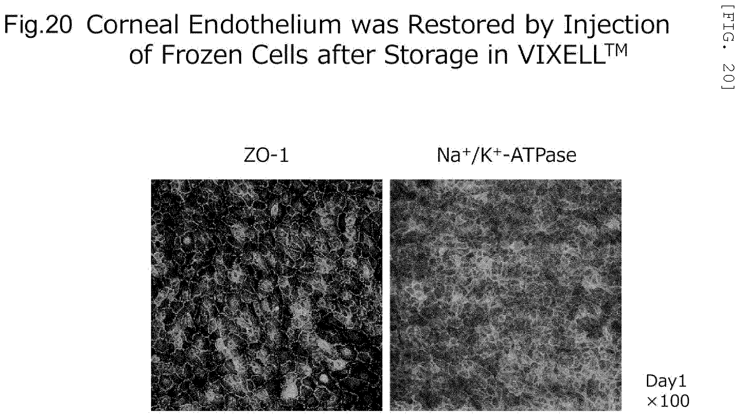

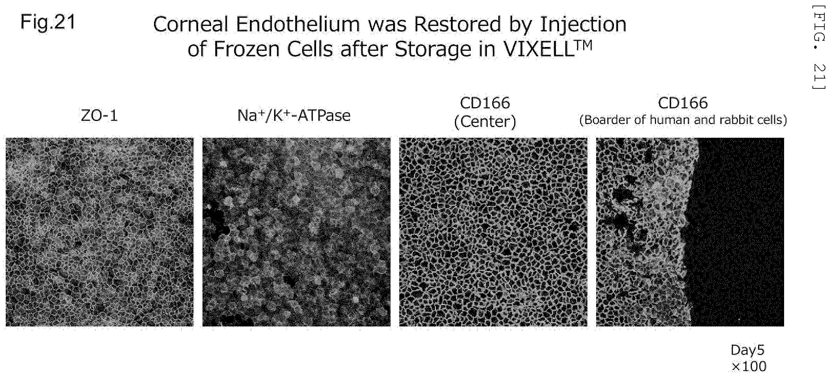

Figure 20 shows photographs of immunohistochemical staining for ZO-1 and Na/K ATPase on day 1 after cell injection. The ZO-1 and Na/K ATPase are expressed as functional molecules in corneal endothelial cells. Figure 21 shows photographs of immunohistochemical staining for CD166, ZO-1, and Na/K ATPase in the corneal endothelium on day 5 after cell injection.

These results indicate that the injected corneal endothelial cells are engrafted and normally function in the corneal endothelium.

### (Example 6: Freezing at cooling rate of -0.7°C/min)

A purpose of this example is to verify viability of corneal endothelial cells when frozen from 4°C at a cooling rate of -0.7°C/min in cryopreservation solutions containing known components as in Example 1 and commercially available preservation solutions as in Example 2.

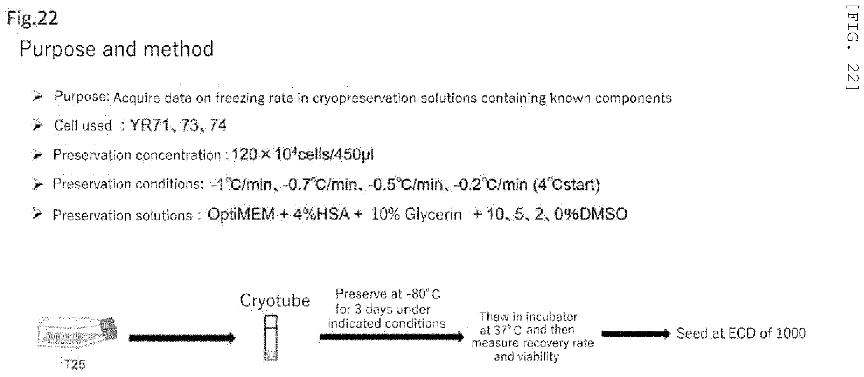

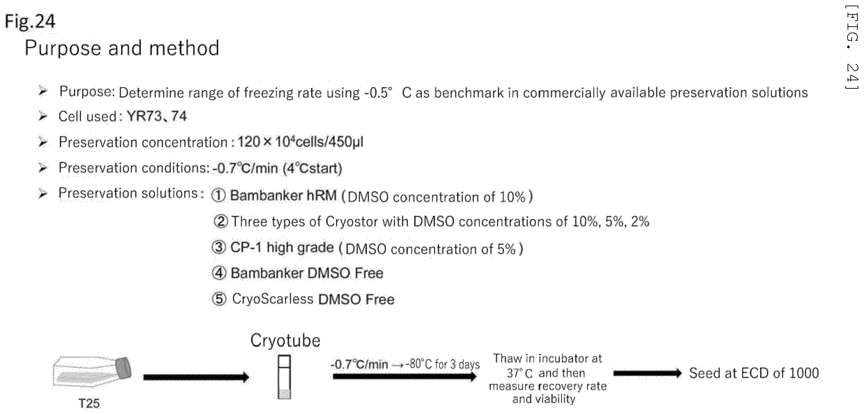

Figures 22 and 24 show an overview of this Example.

### (Results)

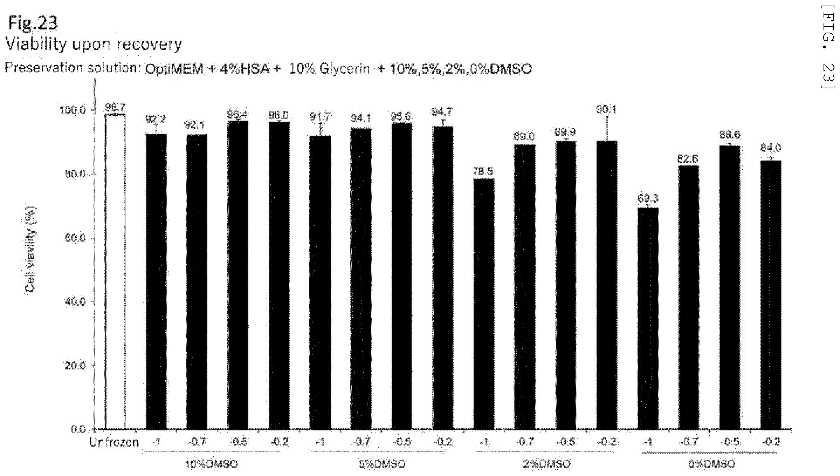

Figure 23 shows a graph comparing cell viability after being frozen in cryopreservation solutions with 4% human serum albumin and 10% glycerin serving as base components and different concentrations of DMSO at a cooling rate of -1°C/min, -0.7°C/min, -0.5°C/min, or -0.2°C/min and thawed. For the cryopreservation solution containing 10% DMSO, a tendency to improve viability was observed when frozen at cooling rates of -0.5°C/min and -0.2°C. For the cryopreservation solution containing 5% DMSO, a tendency to improve viability was observed when frozen at cooling rates of -0.7°C/min, -0.5°C/min, and -0.2°C. The cryopreservation solutions containing 2% DMSO and without DMSO showed significant improvement in viability when frozen at cooling rates of -0.7°C/min, -0.5°C/min, and - 0.2°C.

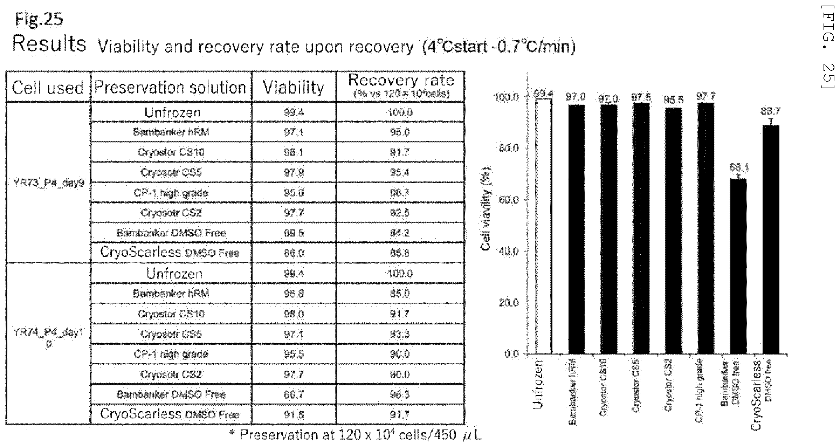

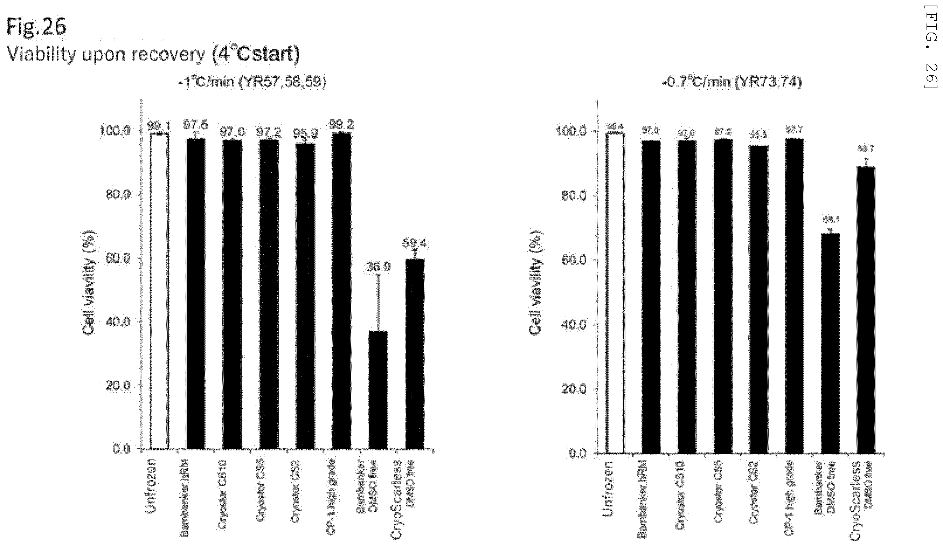

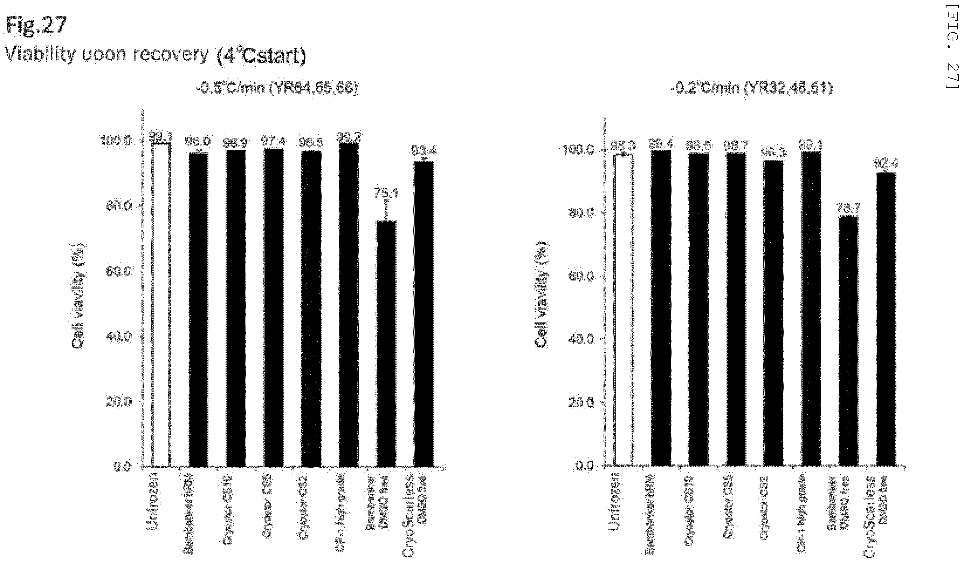

Figure 25 shows cell viability and recovery rate after frozen at a cooling rate of -0.7°C/min from 4°C in commercially available cryopreservation solutions and thawed. The viability was calculated as the number of viable cells/the number of total cells. The cell recovery rate was defined as a recovery rate taking the theoretical cell count (the number of cells filled) as 100%. Figure 26 shows data comparing cell viability after frozen at a cooling rate of -1°C/min or -0.7°C/min from 4°C in commercially available cryopreservation solutions and thawed. Figure 27 shows data comparing cell viability after frozen at a cooling rate of -0.5°C/min or -0.2°C/min from 4°C in commercially available cryopreservation solutions and thawed. For the cryopreservation solutions without DMSO, significant improvement in viability was observed when frozen at cooling rates of-0.7°C/min, -0.5°C/min, and -0.2°C/min compared to when frozen at a cooling rate of -1°C/min.

Based on the results of Examples 1 and 2 and this example, cell viability is improved by freezing at a cooling rate of - 0.7°C/min or slower and preserving. In particular, significant improvement in viability was observed in the cryopreservation solutions containing as low as 2% DMSO and in the cryopreservation solutions without DMSO. Therefore, the method of this disclosure can reduce DMSO upon cryopreservation.

### (Example 7: Cryopreservation in glass vial formulation)

In this example, cryopreservation was performed in glass vials. As shown below, freezing at a slow cooling rate allows for preservation with high viability regardless of container.

### (Material and method)

Cells were collected in the same manner as in Example 2 (Steps 1 to 10), and cells were preserved in glass vials with the following preservation solutions and cooling rates.
Control group: preservation solution: CS2 + Y-27632 (100 µM), freezing rate: -0.5°C/min (to -80°C)
HSA addition group: preservation solution: CS2 + Y-27632 (100 pM) + 4% HSA, freezing rate: -0.5°C/min (to -80°C)
Rate (1) group: preservation solution: CS2 + Y-27632 (100 µM), freezing rate: -0.5°C/min (to -10°C), hold for 110 min (-10°C), -1.0°C/min (to -80°C)
Rate (2) group: preservation solution: CS2 + Y-27632 (100 µM), freezing rate: -0.1°C/min (to -10°C), -1.0°C/min (to - 80°C)

### (Results)

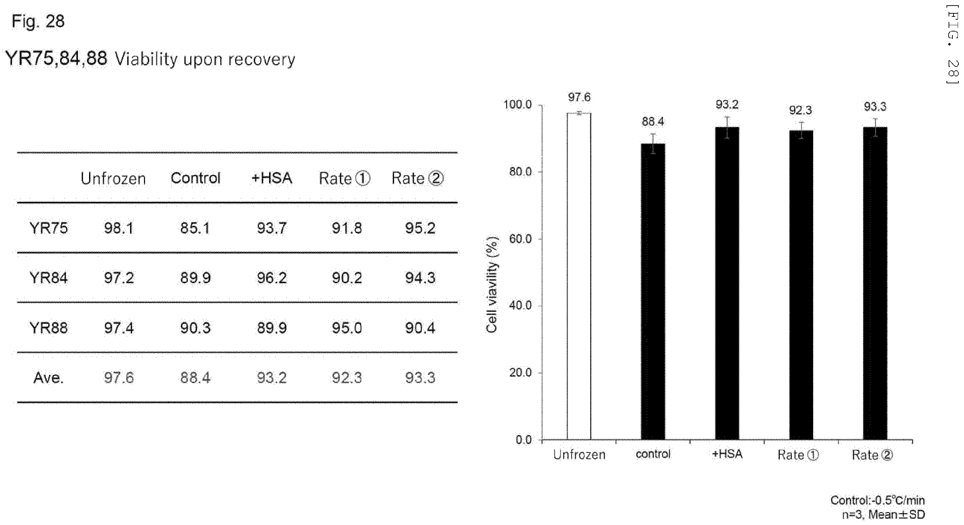

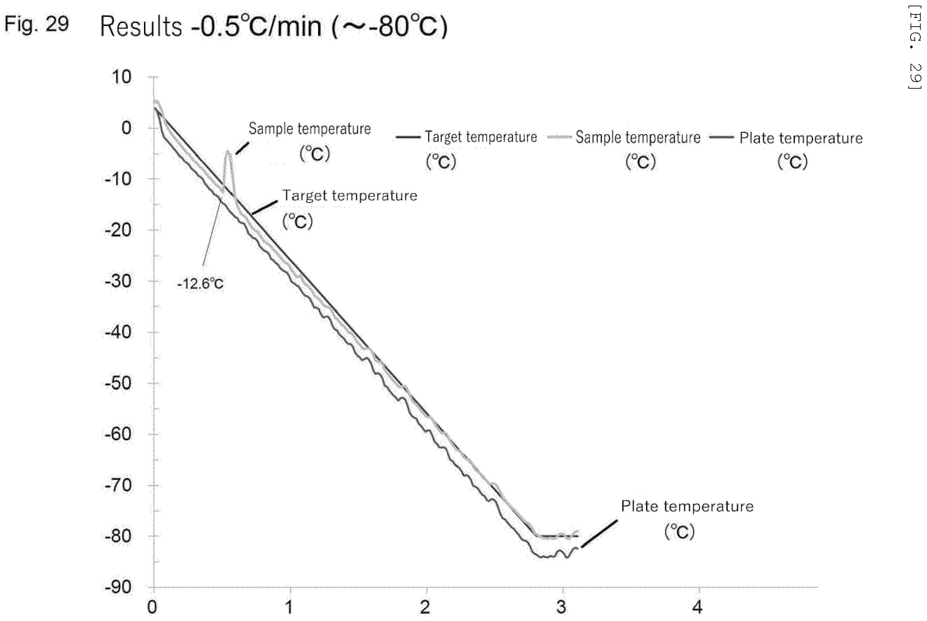

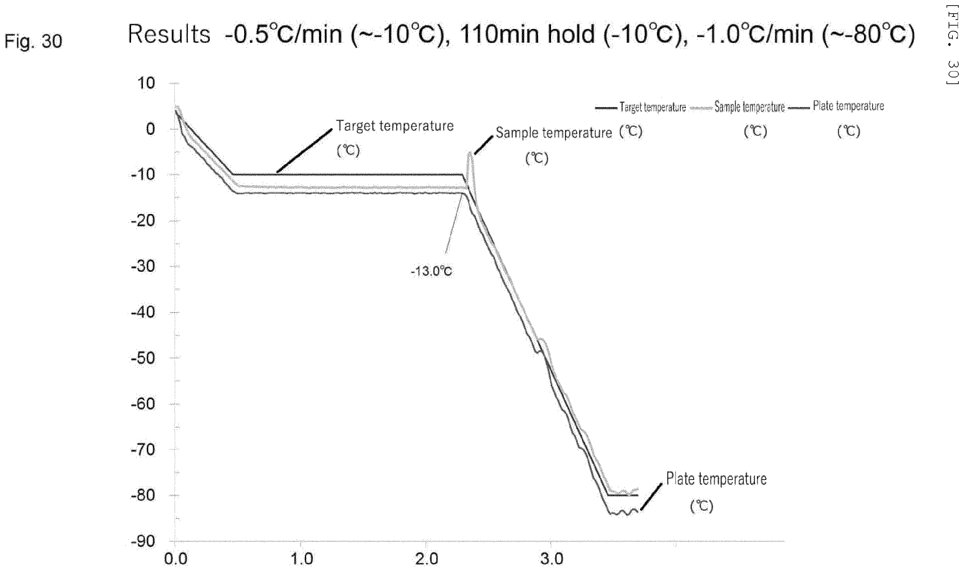

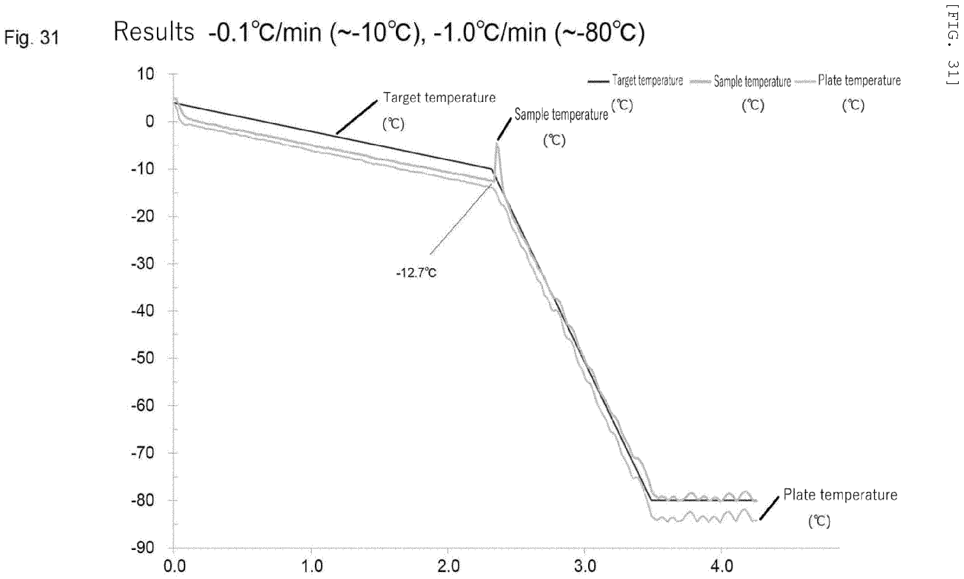

Figures 29 to 31 show temperature transition. A rapid increase in sample temperature is due to latent heat released during cooling. It was shown that a sample was preserved with high viability of higher than 85% when the sample was frozen at -0.5°C/min to-80°C. When the sample was added with HSA and preserved under the same cooling condition, viability was further increased and exceeded 90%. Similarly, when the sample was cooled to -10°C at a slow rate (and then might be maintained at -10°C for a period of time), cooled at a rate of-1.0°C/min, and then preserved, viability was higher than 90% which was higher than that of the control group (Figure 28). No abnormality in cell morphology was observed in all preservation groups.

Thus, it was shown that cells can be preserved with high viability by freezing the cells at a slow cooling rate and preserving them regardless of container. It was also shown that cell viability was further improved by cooling cells to a specific temperature at a slow rate (after which the temperature might be maintained for a period of time) and then cooling the cells at a faster rate and preserving them.

Although the present disclosure has been illustrated above with preferred embodiments of the present disclosure, it is to be understood that the present disclosure should be construed only by the claims. It is to be understood that the patents, patent applications, and references cited herein should be incorporated herein by reference in its entirety as if specifically set forth herein. The present application claims the benefit of priority to Japanese Patent Application No. 2021-184246, filed November 11, 2021, the contents of which are incorporated herein by reference.

### [Industrial Applicability]

Provided is a method for freezing corneal endothelial cells in a cryopreservation solution with a reduced concentration of or free from DMSO and a method for producing a frozen cell formulation that can be administered directly to a patient. Thus, the formulation can be used for cell transplantation and the like and is available in the pharmaceutical field.

## Claims

1. A method for preserving corneal endothelial cells and/or corneal endothelium-like cells, the method comprising:
freezing the corneal endothelial cells and/or the corneal endothelium-like cells in a non-frozen state, the freezing comprising at least one step of decreasing a temperature at a rate of less than 1°C per minute when changing the temperature from a non-frozen temperature to a freezing target temperature; and
optionally maintaining the corneal endothelial cells and/or the corneal endothelium-like cells in a frozen state.

2. The method according to claim 1, comprising maintaining the corneal endothelial cells and/or the corneal endothelium-like cells in a frozen state.

3. The method according to claim 1 or 2, wherein maintaining the corneal endothelial cells and/or the corneal endothelium-like cells in a frozen state comprises maintaining the corneal endothelial cells and/or the corneal endothelium-like cells at a freeze maintenance temperature.

4. The method according to claim 3, wherein the freeze maintenance temperature is a temperature in a range of about - 80°C to about -10°C.

5. The method according to claim 3, wherein the freeze maintenance temperature is a temperature in a range of about - 196°C to about -10°C.

6. The method according to claim 3, wherein the freeze maintenance temperature is a temperature of about -30°C or less.

7. The method according to any one of claims 1 to 6, wherein the corneal endothelial cells and/or the corneal endothelium-like cells are decreased in temperature at a rate of about 0.1°C to about 0.9°C per minute from the non-frozen temperature.

8. The method according to any one of claims 1 to 6, wherein the corneal endothelial cells and/or the corneal endothelium-like cells are decreased in temperature at a rate of about 0.2°C to about 0.8°C per minute from the non-frozen temperature.

9. The method according to any one of claims 1 to 6, wherein the corneal endothelial cells and/or the corneal endothelium-like cells are decreased in temperature at a rate of about 0.7°C per minute or less from the non-frozen temperature.

10. The method according to any one of claims 1 to 6, wherein the corneal endothelial cells and/or the corneal endothelium-like cells are decreased in temperature at a rate of about 0.2°C to about 0.7°C per minute from the non-frozen temperature.

11. The method according to any one of claims 1 to 10, wherein the non-frozen temperature is a temperature in a range of about 0°C to about 42°C.

12. The method according to any one of claims 1 to 10, wherein the non-frozen temperature is a temperature in a range of about 0°C to about 37°C.

13. The method according to any one of claims 1 to 10, wherein the non-frozen temperature is a temperature in a range of about 4°C to about 23°C.

14. The method according to any one of claims 1 to 13, wherein freezing comprises at least one step of decreasing a temperature at a rate of less than 1°C per minute in at least a portion of a temperature range of about -20°C ± 10°C.

15. The method according to any one of claims 1 to 13, wherein freezing comprises at least one step of maintaining a temperature in a range of about -20°C ± 10°C for a period of time or longer.

16. The method according to any one of claims 1 to 15, wherein the corneal endothelial cells and/or the corneal endothelium-like cells are preserved in a preservation solution containing less than about 7% DMSO.

17. The method according to any one of claims 1 to 15, wherein the corneal endothelial cells and/or the corneal endothelium-like cells are preserved in a preservation solution comprising about 5% or less DMSO.

18. The method according to any one of claims 1 to 15, wherein the corneal endothelial cells and/or the corneal endothelium-like cells are preserved in a preservation solution comprising about 2% or less DMSO.

19. The method according to any one of claims 1 to 15, wherein the corneal endothelial cells and/or the corneal endothelium-like cells are preserved in a preservation solution free from DMSO.

20. The method according to any one of claims 1 to 19, wherein freezing comprises freezing the corneal endothelial cells and/or the corneal endothelium-like cells in a presence of a ROCK inhibitor.

21. The method according to claim 1, wherein freezing comprises decreasing a temperature at a first rate to a first target temperature and decreasing the temperature at a second rate from the first target temperature to a second target temperature, the first rate being less than 1°C per minute and slower than the second rate.

22. The method according to claim 21, wherein freezing further comprises decreasing a temperature to the first target temperature and then maintaining the temperature at the first target temperature.

23. The method according to claim 21 or 22, wherein the first target temperature is a temperature in a range of about -20°C to about -5°C.

24. The method according to claim 21 or 22, wherein the first target temperature is a temperature in a range of about -15°C to about -10°C.

25. The method according to any one of claims 21 to 24, wherein the second target temperature is a temperature of about -20°C or less.

26. The method according to any one of claims 21 to 24, wherein the second target temperature is a temperature in a range of about -196°C to about -80°C.

27. The method according to any one of claims 21 to 26, wherein the first rate is a rate of about 0.5°C to about 0.05°C per minute.

28. The method according to any one of claims 21 to 26, wherein the first rate is a rate of about 0.3°C to about 0.1°C per minute.

29. The method according to any one of claims 21 to 28, wherein the second rate is a rate of about 0.5 to about 5°C per minute.

30. The method according to any one of claims 21 to 28, wherein the second rate is a rate of about 1 to about 3°C per minute.

31. A method for producing a frozen formulation of corneal endothelial cells and/or corneal endothelium-like cells, the method comprising:
freezing the corneal endothelial cells and/or the corneal endothelium-like cells in a non-frozen state optionally mixed with a pharmaceutically acceptable component to thereby produce a frozen formulation, the freezing comprising at least one step of decreasing a temperature at a rate of less than 1°C per minute when changing the temperature from a non-frozen temperature to a freezing target temperature; and
optionally maintaining the frozen formulation of the corneal endothelial cells and/or the corneal endothelium-like cells in a frozen state.

32. The method according to claim 31, further comprising one or more features described in the method according to any one or more of claims 2 to 30.

33. A frozen formulation of corneal endothelial cells and/or corneal endothelium-like cells produced by the method according to any one of claims 1 to 32.

34. The frozen formulation according to claim 33, wherein the frozen formulation comprises about 1 × 10⁵ to about 3 × 10⁶ corneal endothelial cells and/or corneal endothelium-like cells.

35. The frozen formulation according to claim 33 or 34, wherein the frozen formulation has a volume of about 50 µL to about 600 µL.

36. The frozen formulation according to any one of claims 33 to 35, wherein the frozen formulation is administered in a volume of about 50 µL to about 350 µL per dose.

37. A device for preserving corneal endothelial cells and/or corneal endothelium-like cells, the device comprising:
a housing/preserver that contains a container for containing the corneal endothelial cells and/or the corneal endothelium-like cells;
a temperature controller that gives an instruction to control a temperature of the corneal endothelial cells and/or the corneal endothelium-like cells in the container contained in the housing/preserver; and
a temperature regulator that is able to regulate the temperature in the housing/preserver based on the instruction from the temperature controller,
the temperature controller being able to give an instruction to control the temperature so as to comprise at least one step of changing the temperature at a rate of less than 1°C per minute when decreasing the temperature from a non-frozen temperature to a freezing target temperature and optionally to maintain the corneal endothelial cells and/or the corneal endothelium-like cells in a frozen state.

38. A program for causing a computer to execute a method for preserving corneal endothelial cells and/or corneal endothelium-like cells in a device, the device comprising:
a housing/preserver that contains a container for containing the corneal endothelial cells and/or the corneal endothelium-like cells;
a temperature controller that gives an instruction to control a temperature of the corneal endothelial cells and/or the corneal endothelium-like cells in the container contained in the housing/preserver; and
a temperature regulator that is able to regulate the temperature in the housing/preserver based on the instruction from the temperature controller,
the program causing the temperature controller to execute:
controlling the temperature so as to comprise at least one step of changing the temperature at a rate of less than 1°C per minute when decreasing the temperature from a non-frozen temperature to a freezing target temperature; and
optionally maintaining the corneal endothelial cells and/or the corneal endothelium-like cells in a frozen state.

39. A recording medium storing a program for causing a computer to execute a method for preserving corneal endothelial cells and/or corneal endothelium-like cells in a device, the device comprising:
a housing/preserver that contains a container for containing the corneal endothelial cells and/or the corneal endothelium-like cells;
a temperature controller that gives an instruction to control a temperature of the corneal endothelial cells and/or the corneal endothelium-like cells in the container contained in the housing/preserver; and
a temperature regulator that is able to regulate the temperature in the housing/preserver based on the instruction from the temperature controller,
the program causing the temperature controller to execute:
controlling the temperature so as to include at least one step of changing the temperature at a rate of less than 1°C per minute when decreasing the temperature from a non-frozen temperature to a freezing target temperature; and
optionally maintaining the corneal endothelial cells and/or the corneal endothelium-like cells in a frozen state.

40. A frozen formulation comprising:
less than 7% DMSO; and
corneal endothelial cells and/or corneal endothelium-like cells.

41. A frozen formulation comprising:
less than 7% DMSO; and
corneal endothelial cells and/or corneal endothelium-like cells,
the frozen formulation being able to be administered directly to an eye after thawing.

42. A frozen formulation comprising:
less than 7% DMSO; and
corneal endothelial cells and/or corneal endothelium-like cells,
the DMSO and the corneal endothelial cells and/or the corneal endothelium-like cells being in a slow frozen state.

43. A frozen formulation comprising:
less than 7% DMSO;
corneal endothelial cells and/or corneal endothelium-like cells; and
a saline component,
the DMSO, the corneal endothelial cells and/or the corneal endothelium-like cells, and the saline component being in a frozen state.

44. A frozen formulation comprising:
less than 7% DMSO;
corneal endothelial cells and/or corneal endothelium-like cells; and
a medium component,
the DMSO, the corneal endothelial cells and/or the corneal endothelium-like cells, and the medium component being in a frozen state.

45. A post-thaw long-term stable frozen cell formulation comprising:
less than 7% DMSO; and
corneal endothelial cells and/or corneal endothelium-like cells.

46. The frozen formulation according to any one of claims 40 to 45, wherein the frozen formulation comprises about 5% or less DMSO.

47. The frozen formulation according to any one of claims 40 to 45, wherein the frozen formulation comprises about 2% or less DMSO.

48. The frozen formulation according to any one of claims 40 to 45, wherein the frozen formulation is free from DMSO.

49. The frozen formulation according to any one of claims 40 to 48, further comprising a ROCK inhibitor.

50. The frozen formulation according to claim 49, wherein the ROCK inhibitor is Y-27632.

51. A frozen formulation comprising:
a ROCK inhibitor; and
corneal endothelial cells and/or corneal endothelium-like cells,
the ROCK inhibitor and the corneal endothelial cells and/or the corneal endothelium-like cells being in a frozen state.

52. A frozen formulation comprising:
a ROCK inhibitor; and
corneal endothelial cells and/or corneal endothelium-like cells; and
a saline component,
the ROCK inhibitor, the corneal endothelial cells and/or the corneal endothelium-like cells, and the saline component being in a frozen state.

53. A frozen formulation comprising:
a ROCK inhibitor;
corneal endothelial cells and/or corneal endothelium-like cells; and
a medium component,
the ROCK inhibitor, the corneal endothelial cells and/or the corneal endothelium-like cells, and the medium component being in a frozen state.

54. A post-thaw long-term stable frozen cell formulation comprising:
corneal endothelial cells and/or corneal endothelium-like cells; and
a ROCK inhibitor.

55. The formulation according to claim 54, wherein the corneal endothelial cells and/or the corneal endothelium-like cells have at least 80% viability for at least 6 hours at room temperature after thawing.

56. A frozen formulation comprising:
corneal endothelial cells and/or corneal endothelium-like cells; and
a ROCK inhibitor,
the corneal endothelial cells and/or the corneal endothelium-like cells and the ROCK inhibitor being in a slow frozen state, and
the formulation not inhibiting engraftment and survival in vivo of the corneal endothelial cells and/or the corneal endothelium-like cells administered after thawing.

57. A frozen formulation comprising:
less than 7% DMSO;
a ROCK inhibitor; and
corneal endothelial cells and/or corneal endothelium-like cells,
the DMSO, the ROCK inhibitor, and the corneal endothelial cells and/or the corneal endothelium-like cells being in a slow frozen state.

58. The formulation of any one of claims 50 to 57, wherein the ROCK inhibitor is Y-27632.

59. The formulation according to any one of claims 50 to 56, wherein the formulation comprises less than about 7% DMSO.

60. The formulation according to any one of claims 50 to 56, wherein the formulation comprises about 5% or less DMSO.

61. The formulation according to any one of claims 50 to 56, wherein the formulation comprises about 2% or less DMSO.

62. The formulation according to any one of claims 50 to 56, wherein the formulation is free from DMSO.

63. The formulation according to any one of claims 30 to 52, wherein the formulation comprises the cells in a slow-frozen state.

64. The formulation according to any one of claims 30 to 53, wherein the formulation is frozen by decreasing a temperature at a rate of less than 1°C per minute from a non-frozen temperature.

65. The formulation according to any one of claims 40 to 64, wherein the corneal endothelial cells and/or the corneal endothelium-like cells are used for a cell infusion therapy.

66. The formulation according to any one of claims 40 to 65, wherein the frozen formulation is administered after thawing without further processing or culturing.

67. The formulation according to any one of claims 40 to 66, wherein the formulation comprises about 1 × 10⁵ to about 3 × 10⁶ corneal endothelial cells and/or corneal endothelium-like cells.

68. The formulation according to any one of claims 40 to 67, wherein the formulation has a volume of about 50 µL to about 600 µL.

69. The formulation according to any one of claims 40 to 68, wherein the formulation is administered in a volume of about 50 µL to about 350 µL per dose.

70. A frozen formulation kit comprising:
a container that contains a frozen formulation comprising corneal endothelial cells and/or corneal endothelium-like cells in a frozen state; and
a housing that contains the container while maintaining it in a frozen state.

71. The frozen formulation kit according to claim 70, wherein the frozen formulation is the formulation according to any one of claims 30 to 59.

72. A frozen formulation kit comprising:
the formulation according to any one of claims 40 to 69;
a container that contains the formulation; and
a housing that contains the container while maintaining the formulation in a frozen state.

73. A frozen formulation kit comprising:
a container; and
a housing that contains the container,
the container being used to contain the formulation according to any one of claims 40 to 69, and the housing being used to maintain the formulation in a frozen state.

74. A frozen formulation kit comprising:
a container that contains a frozen formulation comprising a ROCK inhibitor and corneal endothelial cells and/or corneal endothelium-like cells both in a frozen state; and
a housing that contains the container while maintaining it in a frozen state.

75. A use of a kit, the kit comprising:
a container; and
a housing that contains the container,
the container being used to contain the formulation according to any one of claims 40 to 69, and the housing being used to maintain the formulation in a frozen state.

76. A method for transporting and/or preserving the formulation according to any one of claims 40 to 69, the method comprising:
placing the formulation in a container of a kit comprising the container and a housing that contains the container; and
maintaining the formulation in the kit in a frozen state.

77. A method for performing a corneal endothelial cell injection therapy, the method comprising:
providing corneal endothelial cells and/or corneal endothelium-like cells suitable for the cell infusion therapy;
freezing the corneal endothelial cells and/or the corneal endothelium-like cells, the freezing comprising at least one step of decreasing a temperature of the corneal endothelial cells and/or the corneal endothelium-like cells at a rate of less than 1°C per minute from a non-frozen temperature;
maintaining the corneal endothelial cells and/or the corneal endothelium-like cells in a frozen state and optionally transporting them to the infusion therapy;
thawing the corneal endothelial cells and/or the corneal endothelium-like cells; and
administering the corneal endothelial cells and/or the corneal endothelium-like cells to a subject.
